(19) Europäisches Patentamt
European Patent Office
Office européen des brevets

(11) EP 2 415 749 B1

(12) EUROPEAN PATENT SPECIFICATION

(45) Date of publication and mention of the grant of the patent:
**04.05.2016 Bulletin 2016/18**

(21) Application number: **10758037.5**

(22) Date of filing: **29.03.2010**

(51) Int Cl.:
***C07C 59/52*** (2006.01) ***C07C 51/42*** (2006.01)
***A61K 31/192*** (2006.01) ***A61P 9/00*** (2006.01)

(86) International application number:
**PCT/CN2010/071388**

(87) International publication number:
**WO 2010/111935 (07.10.2010 Gazette 2010/40)**

# (54) NEW SALVIANOLIC ACID COMPOUND L, PREPARATION METHOD AND USE THEREOF

NEUE SALVIANOLSÄUREVERBINDUNG L, ZUBEREITUNGSVERFAHREN UND VERWENDUNG

NOUVEAU COMPOSÉ D'ACIDE SALVIANOLIQUE L, PROCÉDÉ DE PRÉPARATION ET D'UTILISATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.03.2009 CN 200910068289**

(43) Date of publication of application:
**08.02.2012 Bulletin 2012/06**

(73) Proprietor: **Tasly Pharmaceutical Group Co., Ltd.**
**Tianjin 300410 (CN)**

(72) Inventors:
- **ZHOU, Shuiping**
  **Tianjin 300410 (CN)**
- **LI, Wei**
  **Tianjin 300410 (CN)**
- **JIN, Yuanpeng**
  **Tianjin 300410 (CN)**
- **MA, Xiaohui**
  **Tianjin 300410 (CN)**
- **HAN, Jianping**
  **Tianjin 300410 (CN)**
- **CUI, Hongfang**
  **Tianjin 300410 (CN)**
- **LUO, Xuejun**
  **Tianjin 300410 (CN)**
- **CHEN, Xiaopeng**
  **Tianjin 300410 (CN)**

(74) Representative: **Gulde & Partner**
**Patent- und Rechtsanwaltskanzlei mbB**
**Wallstraße 58/59**
**10179 Berlin (DE)**

(56) References cited:
**EP-A1- 1 371 368** **EP-A1- 1 741 439**
**EP-A1- 1 741 439** **CN-A- 1 378 837**
**CN-C- 100 457 748** **US-A- 6 043 276**

- **KELLEY C J ET AL: "Polyphenolic Acids of Lithospermum ruderale Dougl. ex Lehm. (Borginaceae). 1. Isolation and Structure Determination of Lithospermic Acid", JOURNAL OF ORGANIC CHEMISTRY, ACS, US, vol. 40, no. 12, 1 January 1975 (1975-01-01), pages 1804-1815, XP002204923, ISSN: 0022-3263, DOI: 10.1021/JO00900A028**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 415 749 B1

## Description

The present invention relates to the field of traditional Chinese medicine (TCM), more specifically, to a new kind of salvianolic acid compound.

## BACKGROUND OF THE INVENTION

*Radix Salviae Miltiorrhizae* (Chinese crude drug, hereafter named as "Danshen"), the dried root of *Salvia miltiorrhiza Bge.* (*Fam. Labiatae),* is bitter in taste and a little cold, acting on the Channels of heart and liver with the functions of stopping pain by removing stasis, activating blood flow and relieving restlessness by cleaning heart. A series of modern pharmacological investigations have been carried out on Danshen, showing that it has the effects of dilating coronary artery, improving micro-circulation and protecting heart, and is capable of inhibiting and removing platelet aggregation, increasing body's capability of anoxia tolerance and the activities of anti-hepatitis, anti-tumor and anti-virus etc.

In 2001, L.N. Li et al. in Institute of Materia Medica, Chinese Academy of Medical Sciences & Peking Union Medical College, [Bulletin of Medical Research, 2001, Vol. 30(7)] reported that there were 13 water-soluble bioactive components of phenolic acid derivatives isolated from Danshen or the same genus plants, including salvianolic acid A, B, C, D, E, F, G, H, I, J, lithospermic acid, rosmarinci acid and isosalvianolic acid C, etc. In addition, the pharmacological action of these components had also been disclosed.

Rena. Kasimu et al., [Journal of Xinjiang Medical University, 2002, Vol. 25(3)] reported the chemical structure of salvianolic acid K.

Foreign researchers have also studied on water-soluble bioactive components of Danshen. In 1999, George Washington University had applied and finally been granted a US patent with respect to the effect of 13 salvianolic acid derivatives on anti-HIV integrase and other viruses. All of these suggested that Danshen is a medicinal plant resource which has great potential and is worth being developed.

In EP1741439A1 a traditional Chinese medicine preparation based on extracting Danshen and *Notoginseng* is disclosed comprising isolithospermic acid B (see compound 10 on page 4) for the treatment of cardiovascular and cerebrovascular diseases.

In this document, the racemic form of salvionolic acid has been described as one of the several ingredients of a composition for the treatment of cardiocerebral blood vessel diseases.

Said salvianolic acid L of the present invention is just a novel compound that has been found in Danshen in the process of massive screening. Up to now, the structure and pharmacological effects relevant to this compound have not yet been reported.

## SUMMARY OF THE INVENTION

The objective of the present invention is to provide a *trans-form* of salvianolic acid L.

The further objective of the present invention is to provide a pharmaceutical composition comprising the trans-form of salvianolic acid L. Another objective of the present invention is to provide a method for preparing the trans-form of salvianolic acid L.

Another objective of the present invention is to provide a use of the trans-form of salvianolic acid L in the preparation of a medicament for treating cardiovascular diseases.

The present invention relates to a trans-form represented by the general Formula (I) as follows, its pharmaceutically-acceptable salts and solvates:

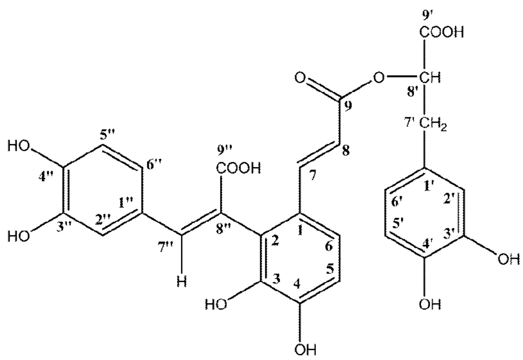

Formula (I)

According to the present invention, the structure of *trans-form* of phenolic acid was identified by physicochemical properties, high resolution mass spectrometry (QFT-ESI), electrospray ionization mass spectrometry (ESI-MS), $^{1}$H-NMR, $^{13}$C-NMR, DEPT, gCOSY, gHMBC and gHMQC.

The compound of the present invention is a pale yellowish powder.

The compound according to the present invention shows a positive result in thin-layer chromatography (TLC) color development reaction with $FeCl_3$, suggested that it may be phenolic compound.

With a high-resolution mass spectrometry (QFT-ESI) which showed a quasi-molecular ion peak at *m/z* 537.1034, the molecular formula was confirmed to be $C_{27}H_{22}O_{12}$ with an unsaturated degree Q of 17.

In ESI-MS, the molecular ion peak of the compound according to the present invention at *m/z* 537 can easily lose 8"-carboxyl group (-44) firstly to form a fragment ion peak at *m/z* 493 (with the same structure as the molecular ion peak of salvianolic acid A), and then form two fragment ion peaks at *m/z* 313, 295 in accordance with the fragmentation regularity of salvianolic acid A.

According to the present invention, the fragmentation regularity of salvianolic acid A is presented as follows:

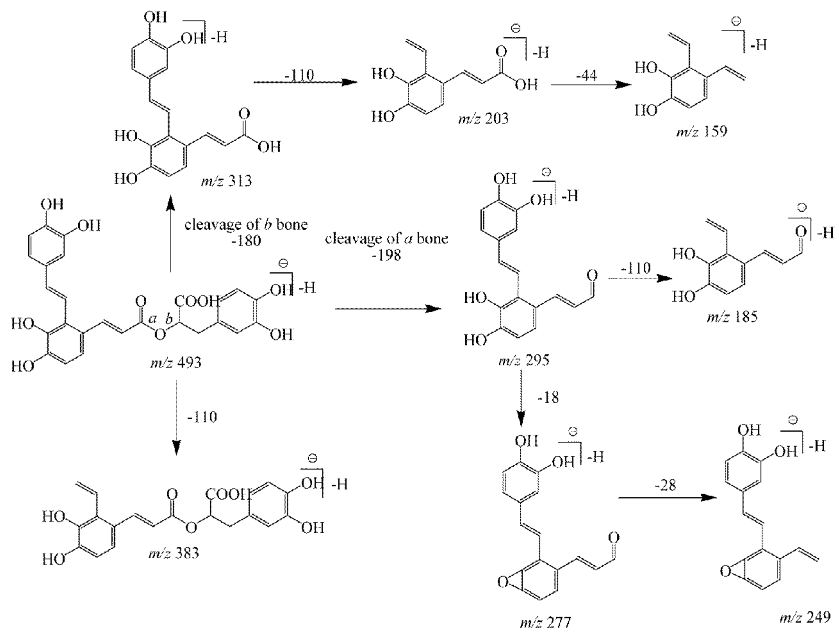


It is clear that the main fragment ion peaks at *m/z* 493, 313, 295 are main ion peaks of salvianolic acid A in its mass-spectrum. Thus, the compound of the present invention has the same backbone structure as that of salvianolic acid A.

Proton nuclear magnetic resonance ($^{1}$H-NMR) spectrum shows 1 signal of methenyl proton attached to oxygen at δ 5.09 (1 H, *dd,* J=8.0, 4.5 Hz); 11 signals of aromatic proton at δ 6.88 (1 H, *d,* J=8.5 Hz), δ 7.25 (1 H, d, J=8.5 Hz), δ 7.59 (1 H, *d,* J=16.0 Hz), δ 6.22 (1 H, *d,* J=16.0 Hz), δ 6.68 (1 H, s), δ 6.55 (2H, *d,* J=8.0 Hz), δ 6.58(1 H, *d,* J=2.0 Hz), δ 6.69(1 H, *d,* J=8.0 Hz), δ 6.54(1 H, *dd,* J=8.5, 2.0 Hz), δ 7.92(1 H, s); 2 signals of aliphatic proton at δ 3.01 (2H, *ddd,* J=14.0, 8.0, 4.5 Hz).

Carbon-13 nuclear magnetic resonance ($^{13}$C-NMR) spectrum shows 27 carbon signals, including 1 aliphatic carbon signal at δ 39.6, 1 signal of methenyl carbon attached to oxygen at δ 76.4, 3 signals of carbonyl carbon at δ 170.1, δ 173.0, δ 175.1 , and 22 signals of double-bond carbon at δ 117.4, δ 117.8, δ 117.8, δ 118.2, δ 119.2, δ 120.2, δ 121.7, δ 123.7, δ 125.7, δ 126.6, δ 128.0, δ 128.8, δ 129.9, δ 130.9, δ 146.2, δ 146.5, δ 146.9, δ 147.4, δ 147.7, δ 147.8, δ 150.3, δ 150.9.

The DEPT spectrum shows that there are $1 \times CH_2$, 12XCH and 14XC in the molecule.

In light of the chemical shift and mutual couple of the aromatic proton in the $^{1}$H-NMR spectrum, together with the information provided by the $^{13}$C-NMR spectrum, the compound of the present invention is considered to have two 1,3,4-tri-substituted benzene rings, one 1,2,3,4-tetra-substituted benzene ring, 1 trans-form double bond and 1 single-substituted double bond. All of these are consistent with the spectrometry characteristics of the compounds of salvianolic acid from *Radix Salviae Miltiorrhizae.*

As a consequence of the above, it could be initially inferred that the compound in the present invention was likely to be a compound of phenolic acid, structurally showing a similarity to the reported compounds of salvianolic acid in *Radix Salviae Miltiorrhizae.*

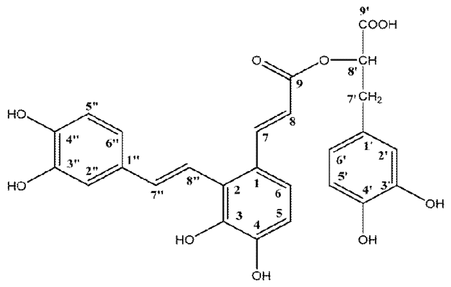

Salvianolic acid A

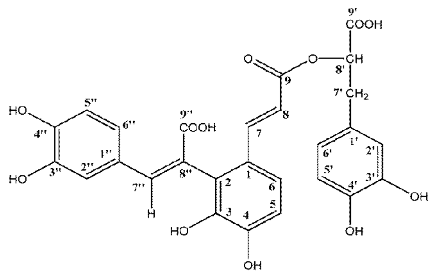

Compound of the present invention

Compared with the prior art and relevant spectrum researches, the compound of the present invention was found to have the similar spectral properties with salvianolic acid A, except that $^{1}$H-NMR showed 2 pairs of trans-form double-bond protons in salvianolic acid A, while just 1 pair of trans-form double-bond proton and 1 single-substituted double-bond proton in the compound of the present invention; and $^{13}$C-NMR shows there is 1 more carbonyl carbon signal in the compound of the present invention than those of salvianolic acid A, meanwhile C-7" and C-8" is shifted downfield respectively by 8ppm and 6ppm. As a result, the difference between the compound in the present invention and salvianolic acid A is that the C-7" or C-8" is substituted by a carboxyl group.

In order to further confirm the substitution of C-7" and C-8", 2D-NMR studies of the present compound were carried out, and its HMBC spectrum results showed that there is a long-range coupling between H-7" and C-9", H-7" and C-2", H-7" and C-2, as well as H-7" and C-6". It therefore could be deduced that C-8" was substituted by a carboxyl group in the present compound.

Accordingly, compared with the prior art, the compound of the present invention is a *trans-form* of salvianolic acid, which is named as the "salvianolic acid L".

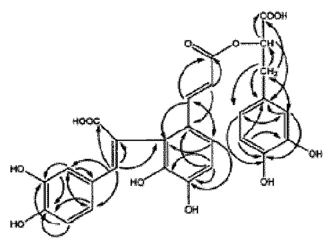

Actually, due to the changes of configuration and conformation that occured in the present compound during the process of extraction, corresponding changes would take place on its spectral data, but various kinds of isomers produced by configurational and conformational changes will fall within the protection scope of the present invention.

The salvianolic acid L of the present invention, according to the ordinary technical knowledge and the prior art, also can be used in the form of its pharmaceutically-acceptable salts or solvates. Said pharmaceutically-acceptable salts of the salvianolic acid L according to the present invention include conventional and pharmaceutically-acceptable salts produced from inorganic or organic base, which are produced by conventional salt-forming method. Suitable examples of the salts include sodium salt, potassium salt, lithium salt, magnesium salt, aluminum salt, calcium salt, zinc salt, or salts formed by reacting with N,N'-dibenzyl ethylenediamine, chloroprocaine, choline, diethanolamine, ethylenediamine, N-methyl glucoseimine, procaine and berberine. The salvianolic acid L described below includes the salvianolic acid L represented by the formula (I) and its pharmaceutically-acceptable salts, solvates,

Said salvianolic acid L of the present invention is appropriately administered in the form of a pharmaceutical composition, which can be used conventionally with one or more kinds of pharmaceutically acceptable carriers or excipients. In addition, if possible, said salvianolic acid L of the present invention can be administered as a raw medicine, preferably the active components directly used as a pharmaceutical preparation. From the viewpoints of compatibility with other components and safety for the patient, the carriers must be pharmaceutically-acceptable.

Therefore, the present invention provides pharmaceutical preparations of the salvianolic acid L, which comprises the salvianolic acid L of the present invention and one or more kinds of pharmaceutically-acceptable carriers, with or without other therapeutical and/or preventative components. These preparations can be administered orally, parenterally (including subcutaneously such as injection or reservoir-type tablet, intra-dermally, intrathecally, intramuscularly such as reservoir-type and intravenously), rectally and topically such as sublingually. The most desirable route of administration, however, depends on the disease of patients. Said pharmaceutical preparations can be a unit preparation, and can be prepared by any method well-known in the pharmaceutical field. All of these methods include the step of combining

the salvianolic acid L of the present invention with a carrier constituting one or more kinds of adjuvant components. Generally speaking, said preparations of the present invention are produced as follows: uniformly and compactly combining the salvianolic acid L of the present invention with fluid, or pulverized solid carries or a mixture thereof to give a semi-product; if necessary, then forming the above semi-product into a desired preparation.

Normally, a series of standard pharmaceutical technologies can be used to give the pharmaceutical composition of the present invention by utilizing the salvianolic acid L and pharmaceutical carries. The technologies include mixing, granulating and pressing. As well-known to the skilled in the art, the characteristics and forms of the pharmaceutically-acceptable carriers or diluents depend on the following factors: the amount of the active components mixed, administration route and other known factors. Said pharmaceutically acceptable carriers herein refer to all sorts of organic or inorganic carriers that can be administered together with the composition, for example, excipient, lubricant, binding agent, disintegrating agent and coating agent used for solid-preparation; or pharmaceutical additives, such as colorant and sweetening-agent. Said pharmaceutical carriers are selected from the group consisting of sugar-alcohol such as mannitol or sorbitol, sodium pyrosulfite, sodium bisulfite, sodium thiosulfate, cysteine hydrochloride, thioglycolic acid, methionine, vitamin C, disodium EDTA, EDTA calcium sodium, carbonates, acetates, phosphates of monovalent alkali metal or their aqueous solutions, hydrochloric acid, acetic acid, sulfuric acid, phosphoric acid, amino acid, sodium chloride, potassium chloride, sodium lactate, xylitol, maltose, glucose, fructose, dextran, glycine, starch, sucrose, lactose, mannitol, silicon derivatives, cellulose and derivatives thereof, alginate, gelatin, polyvinylpyrrolidone (PVP), glycerol, Tween-80, agar, calcium carbonate, calcium bicarbonate, surfactant, PEG, cyclodextrin, β-cyclodextrin, phospholipid materials, kaolin, talc powder, calcium stearate, magnesium stearate etc.

The pharmaceutical composition mentioned above can be formulated into any pharmaceutically-acceptable dosage form, including tablets, such as sugar-coated tablets, film-coated tablets and enteric coated tablets; capsules, such as hard capsules and soft capsules; oral solutions; buccal tablets; granules; granules taken after dissolving in boiling water; pills; powders; pastes; pellets; suspensions; pulvis; liquors; injections; suppositories; pastes, such as ointments and plasters; creams; sprays; drops and patches. Preferably, the preparations are in the oral dosage form, such as capsules, tablets, oral solutions, granules, pills, powders, pellets and pastes; and in the form of injections, such as injectable powders, injections and transfusions etc. Most preferably, the preparations are in the form of tablets.

Among these desirable preparations, said oral preparations can contain commonly-used excipient, binding agent, bulking-agent, diluent, tablet-pressing agent, lubricant, disintegrating agent, colorants, flavoring-agent and wetting-agent, and if necessary, the tablets can be coated.

Preferable examples of said excipient include lactose, D-mannitol, D-sorbitol, starch (such as α-starch), dextrin, crystalline cellulose, low-substituted hydroxypropyl cellulose, sodium carboxymethyl cellulose, arabic gum, amylopectin, light anhydrous silicic acid, synthetic aluminum silicate or magnesium aluminum silicate etc.

Preferable examples of said lubricant include magnesium stearate, calcium stearate, talcum powder and silica gel etc.

Preferable examples of said binding agent include α-starch, sucrose, gelatin, arabic gum, methylcellulose, carboxymethyl cellulose, sodium carboxymethyl cellulose, crystalline cellulose, sugar, D-mannitol, trehalose, dextrin, amylopectin, hydroxypropyl cellulose, hydroxypropyl methyl cellulose, pyrrolidone etc.

Preferable examples of said disintegrating agent include lactose, sugar, starch, carboxymethyl cellulose, calcium carboxymethyl cellulose, aminoalkyl sodium, sodium carboxymethyl starch, light anhydrous silicic acid, low-substituted hydroxypropyl cellulose etc.

Preferable examples of said coating agent include hydroxypropyl methyl cellulose, hydroxypropyl cellulose, ethyl cellulose, carboxymethyl cellulose, polyvinyl alcohol etc.

Preferable examples of said colorant include water-soluble edible tartrazine dye (food dye such as edible red No.2 and No.3, edible yellow No.4 and No.5, edible blue No.1 and No.2); water-insoluble lake colors (such as aluminum salt of the afore-mentioned water-soluble edible tartrazine dye) and natural dye (such as β -carotene, chlorophyll and colcothar) etc.

Preferable examples of said sweetening-agent include saccharin sodium, glycyrrhetinic acid, aspartame and stevioside etc.

Conventional method for preparing tablets comprises combining the salvianolic acid L of the present invention with one or more kinds of pharmaceutically acceptable excipient, and then being pressed or being molded.

Besides, the salvianolic acid L of the present invention can also be formulated into oral liquid preparations, for instance, water-soluble or oil-soluble suspensions, solutions, emulsions, syrups, etc. The salvianolic acid L of the present invention can also be prepared into a dry product, re-blended with water or other suitable carriers before use. This sort of liquid preparations contain conventional additives, including suspending-agent, such as sorbitol syrup, methylcellulose, glucose/syrup, gelatin, hydroxyethyl cellulose, carboxymethyl cellulose, aluminum stearate gel or hydrogenated edible fat; emulsifying-agent, such as lecithin, sorbitan monoleate or arabic gum; non-aqueous carrier (including edible oil), such as almond oil, fractionated coconut oil, butyraceous ester, propylene glycol or ethanol; as well as preservative, such as methyl paraben, nipasol and sorbic acid.

Parenterally-administered preparations include aqueous and non-aqueous sterile injections, optionally, these preparations contain antioxidant, buffering agent, bacteriostatic agent and isotonic agent etc; and the parenterally-administered preparations include aqueous and non-aqueous sterile suspensions, optionally, these preparations contain suspending-agent and thickening agent. Said preparations can be preserved in a single-dose or multi-dose vessel such as sealed ampoules and vials, which can be stored under the freeze drying condition and re-dissolved before use with sterile liquid carrier, for example water for injection.

Rectally-administered preparations can be suppositories containing conventional suppository base, for example, cocoa butter, stearic acid or other glycerides or ethylene glycol.

Oral cavity topically-administered preparations, for example the buccal or sublingually preparations, include troches, wherein the active component is embedded in a flavored base such as sucrose and arabic gum; also pastilles, wherein the active component is embedded in a base such as gelatin and glycerol, or sucrose and arabic gum.

The salvianolic acid L of the present invention is formulated into reservoir-type preparations, such a sustained-release preparation can be administered by implantation (such as subcutaneous implantation or intramuscular implantation) or intramuscular injection. Therefore, the salvianolic acid L of the present invention can be prepared with suitable polymers, hydrophobic materials (for example the emulsion in acceptable oil), or ion-exchange resins, or prepared into a slightly-soluble derivatives, for example the slightly-soluble salt.

According to the ordinary technical knowledge and the prior art, medical effects related to the present invention include prevention and treatment for certain diseases or symptoms. Therapeutically effective amount of the salvianolic acid L of the present invention depends on the property of diseases and individual conditions of patients, or follow the physician's advice. Generally, therapeutically effective amount for adult is in a range of 0.02-5000mg per day, preferably 1-1500mg per day. As described above, the amount can be a single-dosage or multiple-dose that will be taken by patients at appropriate intervals, for example, twice a day, three times a day, four times a day or more. Said preparation of the present invention comprises 0.1-99wt% of active component, preferably 30-95wt% for tablets and capsules; and preferably 3-50wt% for liquid preparations.

The present invention is carried out as follows:

a) extraction: extracting *Radix Salviae Miltiorrhizae* crude drug or a mixture of *Radix Salviae Miltiorrhizae* and other crude drugs with water, adding alcohol to precipitate and obtain a supernatant, then concentrating the supernatant to obtain an extract;

b) separation: dissolving the extract of the step a) in water, applying on the macroporous absorbent resin and then eluting the resin with water to obtain an eluent, acidifying the eluent, applying the acidified eluent again on the macroporous absorbent resin, washing the resin with an acidic aqueous solution to remove impurities and then eluting the resin with ethanol to obtain an ethanol eluent, concentrating the ethanol eluent to obtain an extract;

c) purification: applying the extract of the step b) on the silica gel column by using dried method, isocratic eluting with a mobile phase of chloroform, methanol and formic acid; collecting the eluent; monitoring the whole elution process by TLC, combining characteristically analogous eluents to obtain the salvianolic acid L.

In the step a), said *Radix Salviae Miltiorrhizae* crude drug or a mixture of *Radix Salviae Miltiorrhizae* and other crude drugs can be sliced into decoction pieces, ground into granule or powder, preferably sliced into decoction pieces. Preferably, the root of *Radix Salviae Miltiorrhizae* is used as said *Radix Salviae Miltiorrhizae* crude drug. Said other crude drugs refer to the Chinese crude drugs well known to the skilled in the art, which are compatible with *Radix Salviae Miltiorrhizae,* preferably, *Radix Notoginseng, Radix Astragali* and/or *Radix Polygoni Multiflori.*

In the step a), said water-extraction is as follows: decocting the crude drug with water of 4-8 times the volume of the crude drug, preferably with water 4 times the volume of the crude drug for 1.5-3.5 hours, preferably for 2 hours, filtering; decocting drug residue with water of 3-6 times the volume of the drug residue for 1-3 hours, preferably water of 3 times the volume of the drug residue for 1 hour, filtering; and combining the filtrate, concentrating the filtrate to obtain an extract with a relative density of 1.11-1.28 (80°C), preferably 1.2 (80°C). In order to salify the phenolic acid substances so as to be isolated more easily, an alkali aqueous solution is preferably used in the said water-extraction step, preferably, said alkali is at least one selected from the group consisting of sodium bicarbonate, sodium carbonate, sodium hydroxide, potassium bicarbonate, potassium carbonate and potassium hydroxide, more preferably, sodium bicarbonate or sodium hydroxide. Said alkali aqueous solution is a sodium bicarbonate aqueous solution in a concentration of 0.30%-0.68% or a sodium hydroxide aqueous solution in a concentration of 0.0025‰-0.004‰, preferably, a sodium bicarbonate aqueous solution in a concentration of 0.45%.

In the step a), said alcohol-precipitation is as follows: adding 95% ethanol into the extract to precipitate until the content of the ethanol being 65%-70% (25 °C), preferably being 70%, and standing still for 12-36 hours, preferably for 24 hours; concentrating the supernatant by recovering ethanol under reduced pressure condition, and obtaining an

extract with a relative density of 1.30-1.38 (60°C), preferably 1.37 (60 °C).

In order to better eliminate fat-soluble impurity, an alcohol-extraction is preferably performed before the water-extraction step. In the alcohol-extraction step, decocting twice with 50-95% ethanol of 5-8 times the volume of the crude drug, 1-2 hours each time, filtering, discarding the ethanol-extraction solution and extracting drug residue as the water-extraction mentioned-above.

In the step b), said macroporous resin column can be non-polar or weak polar resin, for example, AB-8, HPD450, HPD700, D101, D4020 or X5, preferably AB-8. The weight ratio of the crude drug to the macroporous absorbent resin is 5:1-1:1, preferably 4:1. The resin column is washed with water of 8-15 times the bed volume, preferably 12 times the bed volume, and thus a water eluent is obtained.

Hydrochloric acid is added into the water eluent to adjust its pH value to 2.2-3.5, preferably 3.0.

Said acidic eluent is applied on the macroporous absorbent resin column again with the weight ratio of the crude drug to the macroporous absorbent resin as 5:1-1:1, preferably 4:1, the column is washed with hydrochloric acid having pH value of 2.2-3.5, preferably 3.0, until the eluent being nearly colorless.

Further, 3-8 times of 50%-95% ethanol is used to wash the column, preferably 4 times of 95% ethanol, and the eluent is concentrated to obtain an extract without alcoholic smell.

In the step c), the extract concentrated in the step b) is dissolved with organic solvent, preferably methanol, mixed with chromatographic silica gel, and preferably, the weight of 200-300 mesh chromatographic silica gel added is equal to the weight of the extract. The well-mixed sample is placed on the well-packed silica gel column, preferably the silica gel packed is 200-300 mesh silica gel, the column is eluted with a mobile phase of chloroform: methanol : formic acid (the volume ratio is: 90:10:3-40:10:0.5), preferably chloroform : methanol : formic acid (the volume ratio is: 85:15:3). Said elution can be an isocratic elution (the ratio of eluent is invariant) or a gradient elution (the ratio of eluent changes with time elapsing). Wherein, said gradient elution can be adjusted according to the polarity of the substance to be collected by using the common knowledge in the art, for example, the polarity of the eluent gradually increased. In order to accurately monitor the elution process, TLC with a developing solvent of chloroform : methanol : formic acid (the volume ratio is: 50:10:2) is preferred.

The characteristically analogous eluents are combined to obtain the salvianolic acid L.

For achieving a better separation effect, a preparative liquid chromatography can be used as a separation tool. For example, the salvianolic acid L is prepared with the following separation conditions: Waters Delta prep 4000 semi-preparative liquid chromatography, column: Agilent Zorbax XDB-C18 (21.2×150mm, 5μm), mobile phase: acetonitrile : 0.1 % formic acid aqueous solution (15 : 85), flow rate: 20ml/min, detection wavelength: 280nm.

As shown in the pharmacodynamic test, the capacity of the salvianolic acid L for scavenging free radical is much greater than that of vitamin C (See Table 3, Fig. 9). Moreover, the reducing capacity of the salvianolic acid L of the present invention is greater than that of vitamin C (See Fig. 10). The salvianolic acid L of the present invention possesses the activities of anti-oxidation and free radical scavenging. As a result, the salvianolic acid L of the present invention can be prepared into a medicine having the activities of scavenging free radical and preventative anti-oxidation function.

Besides, the present invention also relates to a use of the said salvianolic acid L in preparation of medicines for treating cardiovascular diseases. Said cardiovascular disease is at least one selected from the group consisting of hypoxia-induced vasodilatation dysfunction, *in vitro* neuronal injury caused by oxygen deprivation, glucose deprivation and over-oxidation status, and acute myocardial ischemia.

As shown in the pharmacodynamic test of the present invention, the lyophilized powder of the salvianolic acid L can cause a certain right shift of the vasoconstriction curve of norepinephrine, but without significant difference. The lyophilized powder of the salvianolic acid L has significantly enhanced vasodilatation effect on the anoxic vascular ring at three Ach concentrations ($10^{-5}$, $10^{-4}$, $10^{-3}$mol/L) ($P<0.05$). It is illustrated that the salvianolic acid L plays a significant role in improving the hypoxia-caused vasodilatation dysfunction (See Tables 7-8 and Figs. 11-12).

The *trans-form* of salvianolic acid L of the present invention has extensive pharmacological effects on the cardiovascular system, including abatement of the vascular endothelial injury caused by ischemia and hypoxia, promotion of the vascular endothelial hyperplasia, improvement in the myocardial cell injury caused by ischemia and hypoxia, resistance to the atherosclerosis, inhibition of the platelet aggregation and resistance to thrombogenesis. Furthermore, said *trans-form* of salvianolic acid L has effects of dilating the coronary artery, increasing the coronary flow and preventing the injury caused by cerebral ischemia.

As shown in the pharmacodynamic test of the present invention, the *trans-form* of salvianolic acid L of the present invention has a significant improving effect on *in vitro* neural cell injury caused by oxygen deprivation, glucose deprivation and hydrogen peroxide and can increase cell survival rate, and has the function of protecting neuronal cell from oxygen deprivation, glucose deprivation and over-oxidation status (See Tables 12-15). In addition, the *trans-form* of salvianolic acid L of the present invention has an effect of treating acute myocardial ischemia (See Tables 16-17).

## BRIEF DESCRIPTION OF DRAWINGS

Fig. 1 illustrates the high resolution mass spectrogram of the salvianolic acid L.

Fig. 2 illustrates the electrospray ionization mass spectrogram of the salvianolic acid L.

Fig. 3 illustrates the $^1$H-NMR diagram of the salvianolic acid L at 500MHz, by using $CD_3OD$.

Fig. 4 illustrates the $^{13}$C-NMR diagram of the salvianolic acid L at 125MHz, by using $CD_3OD$.

Fig. 5 illustrates the DEPT diagram of the salvianolic acid L at 125MHz, by using $CD_3OD$.

Fig. 6 illustrates the gCOSY diagram of the salvianolic acid L at 500MHz, by using $CD_3OD$.

Fig. 7 illustrates the gHMBC diagram of the salvianolic acid L at 500MHz, by using $CD_3OD$.

Fig. 8 illustrates the gHMQC diagram of the salvianolic acid L at 500MHz, by using $CD_3OD$.

Fig. 9 illustrates the capacity of tested substance for scavenging free radical.

Fig. 10 illustrates the comparison of reducing capacity between the salvianolic acid L and vitamin C.

Fig. 11 illustrates the effect of the salvianolic acid L lyophilized powder on vasoconstriction.

Fig. 12 illustrates the effect of the salvianolic acid L lyophilized powder on vasodilatation.

Fig. 13 illustrates the electrocardiogram (ECG) after treating the pituitary gland with pituitrin, wherein A) is the normal ECG obtained from the model control group, B) is the one obtained from the model control group 15s after being administered with pituitrin, and C) is the one obtained from the model control group 30s after being administered with pituitrin.

## EXAMPLES

The advantageous effects of the salvianolic acid L of the present invention on antioxidation and free radical scavenging are further illustrated by the following specific experimental data.

Unless specified otherwise, the unit % and ‰ mentioned in the present invention represents weight ratio.

### **Example 1** Preparation of the salvianolic acid L

Danshen decoction pieces were placed in an extractor. Water (containing 0.45% sodium bicarbonate) of 4 times the volume of the crude drug was added into the extractor to decoct for 2 hours and filtered. Drug residue was continued to be decocted with water of 3 times the volume of the drug residue for 1 hour and filtered, the filtrate was combined and concentrated to obtain an extract with a relative density of 1.2 (80°C). A 95% ethanol was added into the extract to perform precipitation until the final ethanol content being 70% (25°C), standing still for 12 hours or more. The ethanol was recovered under reduced pressure condition to obtain an extract with a relative density of 1.37 (60°C).

The afore-obtained extract was dissolved with water, and then applied on AB-8 macroporous absorbent resin column, and the column was eluted with water 12 times the bed volume to obtain a water eluent. The pH of the water eluent was adjusted with hydrochloric acid to pH 3.0. Again, the acidified water eluent was applied on AB-8 macroporous absorbent resin column. The acidic aqueous solution with pH-value of 3.0 was used to wash the column until the eluent became nearly colorless. Further, 95% ethanol having a volume of 4 times of the bed volume was used to elute and give the eluent, and then the eluent was concentrated to obtain a thick extract without alcoholic smell.

The resulting extract was dissolved with methanol, in which 200-300 mesh chromatographic silica gel is added and mixed, and the weight of the chromatographic silica gel added is equal to the weight of the extract. The mixed sample was placed on a well-packed silica gel column, and the column is eluted with a mobile phase of chloroform : methanol : formic acid (the volume ratio is: 85:15:3). TLC was used to monitor the whole elution process, and the characteristically analogous eluents were combined to obtain the salvianolic acid L.

By using a high-resolution mass spectrometry (QFT-ESI), a quasi-molecular ion peak was $[M-H]^+$ m/z 537.1034.

Table 1 $^1$H (500M, $CD_3OD$) and $^{13}$C-NMR (125M, $CD_3OD$) data assignment for the salvianolic acid L

| No. | $\delta_H$ | $\delta c$ | H-H COSY | C-H COSY |
|---|---|---|---|---|
| 1 | - | 128.0 | | H-5, H-8 |
| 2 | - | 128.8 | | H-6, H-7, H-7" |
| 3 | - | 146.2 | | H-5 |
| 4 | - | 150.9 | | H-5, H-6 |
| 5 | 6.88(1H, d, J=8.5 Hz) | 117.8 | H-6 | |
| 6 | 7.25(1H, d, J=8.5 Hz) | 121.7 | H-5 | H-7 |
| 7 | 7.59(1H, d, J=16.0 Hz) | 147.4 | H-8 | H-6 |
| 8 | 6.22(1H, d, J=16.0 Hz) | 117.4 | H-7 | |
| 9 | - | 170.1 | | H-7, H-8 |
| 1’ | - | 130.9 | | H-2’, H-5’, H-8’, H-7’ |
| 2’ | 6.68(1H,s) | 119.2 | | H-6’, H-7’ |
| 3’ | - | 146.9 | | H-5’ |
| 4’ | - | 147.8 | | H-2’, H-5’, H-6’ |
| 5’ | 6.55(1H, d, J=8.0 Hz) | 117.8 | | |
| 6’ | 6.55(1H, d, J=8.0 Hz) | 123.7 | | H-2’ |
| 7’ | 3.01 (1H, ddd, J=14.0, 8.0, 4.5 Hz) | 39.6 | H-8’ | H-2’ |
| 8’ | 5.09(1H, dd, J=8.0, 4.5 Hz) | 76.4 | H-7’ | H-7’ |
| 9’ | - | 175.11 | | H-7’, H-8’ |
| 1 " | - | 129.9 | | H-2" |
| 2" | 6.58(1H, d, J=2.0 Hz) | 120.2 | | H-6", H-7" |
| 3" | - | 147.7 | | H-2", H-5" |
| 4" | - | 150.3 | | H-6", H-2" |
| 5" | 6.69(1H, d, J=8.0 Hz) | 118.2 | | |
| 6" | 6.54(1H, dd, J=8.5, 2.0 Hz) | 126.6 | | H-2", H-7" |
| 7" | 7.92(1H, s) | 146.5 | | |
| 8" | - | 125.7 | | H-7" |
| 9" | - | 173.0 | | H-7",H-8" |

DEPT spectrum showed there was 1 $\times CH_2$, 12$\times$CH and 14$\times$C in the molecule.

**Example 2** Preparation of the salvianolic acid L

Danshen and Sanqi decoction pieces were placed in an extractor. Water (containing 0.45% sodium bicarbonate) of 6 times the volume of the crude drug was added into the extractor to decoct for 3 hours and filtered. Drug residue was continued to be decocted with water of 5 times the volume of the drug residue for 2 hours and filtered, the filtrate was combined and concentrated to obtain an extract with a relative density of 1.25 (80°C). A 95% ethanol was added into the extract to perform precipitation until the final ethanol content being 68% (25°C), standing still for 12 hours or more. The ethanol was recovered under reduced pressure condition to obtain an extract with a relative density of 1.32 (60°C).

The afore-obtained extract was dissolved with water, and then applied on AB-8 macroporous absorbent resin column, and the column was eluted with water of 12 times the bed volume to obtain a water eluent. The pH of the water

eluent was adjusted with hydrochloric acid to pH 2.5. Again, the acidified water eluent was applied on AB-8 macroporous absorbent resin column. The acidic aqueous solution with pH-value of 3.0 was used to wash the column until the eluent became nearly colorless. Further, a 95% ethanol of 5 times the bed volume was used to elute and give an eluent, and the eluent was concentrated to obtain a thick extract without alcoholic smell.

The resulting extract was dissolved with methanol, in which 200-300 mesh chromatographic silica gel is added and mixed, and the weight of the chromatographic silica gel added is equal to the weight of the extract. The mixed sample was placed on a well-packed silica gel column, and the column is eluted with a mobile phase of chloroform : methanol : formic acid (the volume ratio is: 85:15:3). TLC was used to monitor the whole elution process, and the characteristically analogous eluents were combined to obtain the salvianolic acid L.

By using a high-resolution mass spectrometry (QFT-ESI), a quasi-molecular ion peak was $[M-H]^+$ m/z 537.1027.

Table 2 $^1H$ (500M, $CD_3OD$) and $^{13}C$-NMR (125M, $CD_3OD$) data assignment for the salvianolic acid L

| No. | $\delta_H$ | $\delta c$ | H-H COSY | C-H COSY |
|---|---|---|---|---|
| 1 | - | 128.0 | | H-5, H-8 |
| 2 | - | 128.8 | | H-6, H-7, H-7" |
| 3 | - | 146.2 | | H-5 |
| 4 | - | 150.9 | | H-5, H-6 |
| 5 | 6.88(1H, d, J=8.5 Hz) | 117.8 | H-6 | |
| 6 | 7.24(1H, d, J=8.5 Hz) | 121.8 | H-5 | H-7 |
| 7 | 7.58(1H, d, J=16.0 Hz) | 147.4 | H-8 | H-6 |
| 8 | 6.20(1H, d, J=16.0 Hz) | 117.4 | H-7 | |
| 9 | - | 170.1 | | H-7, H-8 |
| 1’ | - | 130.9 | | H-2’, H-5’, H-8’, H-7’ |
| 2’ | 6.68(1H, s) | 119.1 | | H-6’, H-7’ |
| 3’ | - | 146.9 | | H-5’ |
| 4’ | - | 147.8 | | H-2’, H-5’, H-6’ |
| 5’ | 6.54(1H, d, J=8.0 Hz) | 117.8 | | |
| 6’ | 6.54(1H, d, J=8.0 Hz) | 123.7 | | H-2’ |
| 7’ | 2.97(1H, ddd, J=14.0, 8.0, 4.0 Hz) | 39.6 | H-8’ | H-2’ |
| 8’ | 5.05(1H, dd, J=8.0, 4.0 Hz) | 76.4 | H-7’ | H-7’ |
| 9’ | - | 175.2 | | H-7’, H-8’ |
| 1" | - | 129.8 | | H-2" |
| 2" | 6.56(1 H, s) | 120.1 | | H-6", H-7" |
| 3" | - | 147.7 | | H-2", H-5" |
| 4" | - | 150.3 | | H-6", H-2" |
| 5" | 6.67(1H, d, J=8.5 Hz) | 118.2 | | |
| 6" | 6.48(1H, d, J=8.5 Hz) | 126.7 | | H-2", H-7" |
| 7" | 7.90(1 H, s) | 146.5 | | |
| 8" | - | 125.7 | | H-7" |
| 9" | - | 173.0 | | H-7", H-8" |

DEPT spectrum showed there was 1 $\times CH_2$, 12$\times$CH and 14$\times$C in the molecule.

**Example 3** Preparation of the salvianolic acid L

Danshen decoction pieces were placed in an extractor. 85% ethanol of 6 times the volume of the crude drug was added into the extractor to decoct twice, 2 hours for each time, and filtered. The ethanol-extraction solution was discarded.

Drug residue was decocted with water (containing 0.45% sodium bicarbonate) of 4 times the volume of the drug residue for 2 hours and filtered, and the drug residue was continued to be decocted with water of 3 times the volume of the drug residue for 1 hour and filtered. The filtrates were combined and concentrated to obtain an extract with a relative density of 1.2 (80°C). A 95% ethanol was added into the extract to perform precipitation until the final ethanol content being 70% (25°C), standing still for 12 hours or more. The ethanol was recovered under reduced pressure condition to obtain an extract with a relative density of 1.37 (60°C).

The afore-obtained extract was dissolved with water, and then applied on AB-8 macroporous absorbent resin column, and the column was eluted with water of 12 times the bed volume to obtain a water eluent. The pH of the water eluent was adjusted with hydrochloric acid to pH 3.0. Again, the acidified water eluent was applied on AB-8 macroporous absorbent resin column. The acidic aqueous solution with pH-value of 3.0 was used to wash the column until the eluent became nearly colorless. Further, a 95% ethanol of 4 times the bed volume was used to elute and give an eluent, and the eluent was concentrated to obtain a thick extract without alcoholic smell.

The resulting extract was dissolved with methanol, in which 200-300 mesh chromatographic silica gel is added and mixed, and the weight of the chromatographic silica gel added is equal to the weight of the extract. The mixed sample was placed on a well-packed silica gel column, and the column is eluted with a mobile phase of chloroform : methanol : formic acid (the volume ratio is: 85:15:3). TLC was used to monitor the whole elution process, and the characteristically analogous eluents were combined to obtain the salvianolic acid L.

**Example 4** Preparation of tablets of the salvianolic acid L

| Formulation: | |
|---|---|
| salvianolic acid L | 100g |
| microcrystalline cellulose | 50g |
| lactose | 50g |
| starch | 51g |
| sodium carboxymethyl starch | 12g |
| 5% PVP anhydrous ethanol | proper amount |
| magnesium stearate | 3g |

The above formulation was prepared into 1000 tablets.

Preparation process:

1. Granulation

The salvianolic acid L and other adjuvants listed in the formulation were sieved through a 100-mesh sieve, respectively. According to the formulation dosage, the salvianolic acid L, microcrystalline cellulose, starch and sodium carboxymethyl starch were well blended by using equivalent progressively increasing method. A proper amount of 5% PVP anhydrous ethanol was used to produce the soft materials, granulated with a 14-mesh sieve and dried at 50-60°C for 1 hour. The magnesium stearate according to the formulation dosage was added to sieve the granule with 14-mesh sifter.

2. Tablet pressing

The resulting granule was pressed with a specific diamond-shaped punch die to prepare the tablets.

**Example 5** Preparation capsules of the salvianolic acid L

| Formulation: | |
|---|---|
| Salvianolic acid L | 100g |
| Starch | 200g |
| Sodium carboxymethyl starch | 12g |
| 5% PVP anhydrous ethanol | proper amount |
| Magnesium stearate | 3g |

The above formulation was prepared into 1000 capsules.

Preparation process:

1. Granulation

The salvianolic acid L and other adjuvants listed in the formulation were sieved through a 100-mesh sieve, respectively. According to the formulation dosage, the salvianolic acid L, starch and sodium carboxymethyl starch were well blended according to equivalent progressively increasing method. A proper amount of 5% PVP anhydrous ethanol was used to produce the soft materials, granulated with a 14-mesh sieve and dried at 50-60°C for 1 hour. The magnesium stearate according to the formulation dosage was added to sieve the granule with 14-mesh sieve.

2. Encapsulation

The resulting granule was loaded into capsules.

**Example 6** Preparation injections of the salvianolic acid L

| Formulation: | |
|---|---|
| Salvianolic acid L | 100g |
| Mannitol | 100g |
| Water for injection | up to 2500 ml |

The above formulation was prepared into1000 units.

Preparation process:

The salvianolic acid L was taken, dissolved with 1000ml of water for injection and stirred uniformly. The mannitol was dissolved with 500ml of water for injection and added into the aforesaid salvianolic acid L solution, stirred uniformly, into which 0.5g of activated carbon was added to stir at an invariant temperature for 20min and filtered. The pH of the filtrate was adjusted to 4.5-5.0, diluted with water for injection to 2500ml, filtered aseptically, loaded separately to obtain the product.

**Example 7** Preparation of the salvianolic acid L lyophilizedpowder

| Formulation: | |
|---|---|
| Salvianolic acid L | 100g |
| Mannitol | 100g |
| Water for injection | 2000 ml |

The above formulation was prepared into 1000 units.

Preparation process:

The salvianolic acid L and mannitol were weighed and dissolved with 1500ml of water for injection by stirring, into which 0.5g of activated carbon was added for decolorization by stirring for 20min, the solution was filtered through microvoid filter film (0.45μm) to remove the carbon and diluted with water for injection up to 2000ml. The resulting solution was filtered aseptically, packed separately and freeze dried to obtain the product.

**PHARMACODYNAMIC EXAMPLES**

Pharmacodynamic example 1 Free-radicals-trapping reaction of the salvianolic acid L

It is believed that free radicals are one kind of highly active substances. They can be produced successively during metabolic processes of cells. Due to their direct or indirect oxidation effect, the free radicals have been shown to take part in physiological and pathological process widely. In the presence of excess amount of free radicals, they always attack macromolecules in the body by oxidation, such as nucleic acid, protein, saccharide and lipid etc. By making these substances denaturation by oxidation, cross-linked and broken, the free radicals cause damages to cell structure and function, resulting in tissue destructions and degenerative alterations of the body. As shown by numerous studies, the free radicals contribute to the pathological processes of a lot of diseases, thus inducing many diseases, such as cardiovascular diseases, some cancers, senile cataract and macular degeneration, some inflammations and diversified types of neuron diseases.

Chemical structural analysis shows: the salvianolic acid compounds are donors of phenolic hydroxyl group, having the structural basis for their antioxidant activity. In this study, 1,1-diphenyl-2-picryl-hydrazyl (DPPH) free-radical scavenging reaction model has been used to observe the free-radical scavenging activity of the salvianolic acid L.

1. Reagents and apparatus

The Salvianolic acid L with a purity of more than 95%, which was provided by Tianjin Tasly Group Academy, was prepared in accordance with the method of example 1.

Vitamin C and DPPH were purchased from SIGMA Inc.

Ultraviolet spectrophotometer (UV-1800) was purchased from Beijing Rayleigh Analytical instrument Co., Ltd.

2. Experimental methods

The total reaction volume was 2ml. 1 ml of the sample solutions at different concentrations in 80% methanol (v/v) were added into 100μM of DPPH methanol solution, mixed uniformly to allow the solution to react for 20min at 25°C in the dark. Absorbance of the reaction solution was measured at 517nm. In this study, vitamin C was regarded as a positive control. Free-radical scavenging rate was calculated in accordance with the following equation:

$$\text{Free-radical scavenging rate } (\%)=[1-A_{sample}/A_{control}) \,/\, A_{control}]\times 100\%$$

Wherein, the $A_{sample}$ means the absorbance of the tested samples, and $A_{control}$ means the absorbance of blank control.

3. Experimental results

Table 3 and Fig. 9 show the DPPH free-radical scavenging rates of the salvianolic acid L and vitamin C at different concentrations. The salvianolic acid L had a much higher free-radical scavenging rate than that of the vitamin C.

Table 3 Comparing of the DPPH free-radical scavenging rate between the salvianolic acid L and vitamin C at different concentrations

| Sample ( μg/ml) | 0.314 | 0.625 | 1.25 | 2.5 | 5 |
|---|---|---|---|---|---|
| Salvianolic acid L | 5.41±0.74 | 12.95±2.42 | 25.21±1.82 | 44.19±3.70 | 83.17±4.12 |
| Vitamin C | 3.42±0.42 | 7.06±1.88 | 13.82±1.83 | 29.39±5.92 | 55.34±7.21 |

Pharmacodynamic example 2 Determination of reducing capacity of the salvianolic acid L

To a certain extent, a potential for preventative antioxidation is represented by the reducing capacity of the drug. The study had been carried out on reducing capacity of the salvianolic acid L of the present invention.

1. Reagents and apparatus

The salvianolic acid L with a purity of more than 95%, which was provided by Tianjin Tasly group Academy, was prepared in accordance with the method of example 1.
Analytically pure potassium ferricyanide was purchased from Tianjin No.1 Chemical Reagent Factory.
Analytically pure trichloroacetic acid was purchased from Sinopharm Chemical Reagent Co., Ltd.
Analytically pure ferric chloride was purchased from Tianjin Fengchuan Chemical Reagent Science and Technology Co., Ltd.
Vitamin C was purchased from SIGMA Inc.
Ultraviolet spectrophotometer (UV-1800) was purchased from Beijing Rayleigh Analytical Instrument Co., Ltd.
Refrigerated centrifuge (Z323K) was purchased from HEMMLE, German.

2. Experimental methods

0.5 ml of 200mM phosphate buffer (pH6.8) containing different concentrations of the salvianolic acid L and 1.0% potassium ferricyanide solution were sucked respectively and cooled on an ice bath after being heated on a water bath (50°C) for 20 min. 0.5ml of trichloroacetic acid solution (10%) was added and centrifuged at 1000g/min for 10 min. 1.0 ml of the resulting supernatant was taken, into which 1.0 ml of distilled water and 0.2 ml of ferric chloride solution (0.1 %) were added, stood still for 10 min and the absorbance was measured at 700nm. Meanwhile, the blank experiment was carried out. Vitamin C is a strongly reducing substance, acting as positive control in this study. Reducing capacity of the sample is represented by subtracting the absorbance of the blank control from the absorbance of the tested sample. Thus, it means the higher absorbance, the stronger reducing capacity.

3. Experimental results

As shown in FIG. 10, both substances had a concentration-dependent absorbance, and reducing capacity of the salvianolic acid L was much stronger than that of vitamin C.

Determination of components and preparation of No.1 extract and No.2 extract used in the following pharmacodynamic examples 3-5.

All the materials used in the experiment were provided by Tianjin Tasly Group Academy TCM Institute. Content of No.1 extract was 6.825g of crude drugs/g No.1 extract, and No.2 extract was 4.162g of crude drugs/g No.2 extract.

Preparation process

Preparation process of No.1 extract:

A mixture of 89.8 wt% *Radix Salviae Miltiorrhizae* (Chinese name: Danshen) and 9.6 wt% *Radix notoginseng* (Chinese name: Sanqi) was extracted with water (containing 0.45% sodium bicarbonate) twice: 5 times of water for 2 hours and 4 times of water for 1 hour in succession. 95% ethanol (v/v) was used to concentrate the water-extraction solution by means of reflux. The ethanol-precipitation was performed until the final ethanol content in the ethanol-extraction solution being 70%. After standing still overnight, a supernatant was taken and concentrated to give the No.1 extract.

Preparation process of No.2 extract:

A mixture of 89.8 wt% *Radix Salviae Miltiorrhizae* (Chinese name: Danshen) and 9.6 wt% *Radix notoginseng* (Chinese name: Sanqi) was extracted with water twice: 5 times of water for 2 hours and 4 times of water for 1 hour in succession. 95% ethanol (v/v) was used to concentrate the water-extraction solution by means of reflux, the ethanol-precipitation was performed until the final ethanol content in the ethanol-extraction solution being 70%. After standing still overnight, a supernatant was taken and concentrated to give the No.2 extract.
Salvianolic acid L was prepared by the method of example 1 of the present invention.

Detection method

Analytical conditions were as follows: Waters 2695 HPLC, Agilent Zorbax SB-C18 (4.6mm×250mm, 5μm) chromatographic column, a 0.02% phosphoric acid aqueous solution was used as mobile phase A and 80%(v/v) acetonitrile solution containing 0.02% phosphoric acid was used as mobile phase B, a gradient elution was performed according to the following Table 4, a flow rate was 1ml/min, a detection wavelength was 280nm, a column temperature was 30°C, and a recording time was 50min.

Table 4 The linear gradient elution table of mobile phases

| Time (min) | mobile phase A | mobile phase B |
|---|---|---|
| 0 | 90 | 10 |
| 8 | 78 | 22 |
| 15 | 74 | 26 |
| 35 | 61 | 39 |
| 40 | 90 | 10 |
| 50 | 90 | 10 |

Content of each component in No.1 and No.2 extracts was presented in the following Table 5 and Table 6.

Table 5 Content of each component in No.1 extract

| Component | Content (%) | Remarks |
|---|---|---|
| Protocatechuic aldehyde | 0.33 | highest peak |
| Danshensu | 0.30 | |
| Salvianolic acid A | 0.17 | |
| Salvianolic acid B | 0.30-0.76 | |
| Salvianolic acid L | 0.43-0.49 | |

Table 6 Content of each component in No.2 extract

| Component | Content (%) | Remark |
|---|---|---|
| Protocatechuic aldehyde | 0.14 | |
| Danshensu | 0.10 | |
| Salvianolic acid A | 0.12 | |
| Salvianolic acid B | 3.5 | |
| Salvianolic acid L | 0 | |

<u>Pharmacodynamic example 3 Effect of the salvianolic acid L lyophilized powder on isolated rat thoracic aorta</u>

Experimental materials

1. Test materials and reagents: Salvianolic acid L lyophilized powder was provided by Tianjin Tasly Group Academy TCM Institute. Norepinephrine Citrate (NA) and acetylcholine (ACH) were purchased from Sigma Inc. with the batch No. of 1377511 44908131. Raw materials for preparing Kreb's solution included: potassium chloride, sodium chloride, potassium dihydrogen phosphate, sodium bicarbonate, magnesium sulfate, glucose and calcium chloride.

2. Main apparatus: MedLab® isolated tissue trough and Medlab-U/8C acquisition system were produced by Nanjing Medease Science and Technology Co., Ltd. Other devices included tension transducer, digital-controlled super thermostatic bath SC-15, analytical balance, water purifier, and oxygen cylinder.

3. Experimental animals: SD rats, male or female in proper body-weight, were provided by Beijing Vital River Laboratory Animal Technology Co., Ltd., with the certificate No. of SCXK (Jing) 2007-0001. All rats were fed with rat special diet (produced by Beijing Keaoxieli Diet Co., Ltd.) and tap water in animal feeding room at a room temperature of 20-25°C, illuminated for 12 hours.

Experimental methods

1. Design of administration dose

Dose of the salvianolic acid L lyophilized powder was confirmed on the basis of pharmacodynamic experiments of other salvianolic acids. In this research, the dose was at 0.1 mg/ml.

Kreb's solution (mol/L): NaCl (120), $NaHCO_3$ (25), $KH_2PO_4$ (1.2), $MgSO_4$(1.2), KCl (4.5), $CaCl_2$ (1.25) , $C_6H_{12}O_6$ (Glucose 11.1)

KCl : 100μl of KCl solution (3mol/L) was added each time (final concentration of 60mmol/L).

NA: $10^{-4}$mol/L (final concentration at $19^{-6}$mol/L), was diluted with totally 4 gradients.

ACH: $10^{-3}$mol/L (final concentration at $10^{-5}$mol/L) was diluted with totally 4 gradients.

2. Grouping

Rats received free diet that was placed in group randomly according to the preparation of drug on the day. It made certain that there were 8 rats in each group and 4 vascular ring data available from each rat. In this research, the rats were divided into 3 groups: normal group, hypoxia model group and the salvianolic acid L + hypoxia model group.

3. Experimental methods

SD rats received free diet that was placed in group randomly according to the preparation of drug on the day, and there were 8 rats in each group. The rats were sacrificed by dislocation of cervical vertebra, and the chest was opened rapidly to take out thoracic aorta. At 0°C, the thoracic aorta was placed into the oxygen-blowing Kreb's solution, where the connective tissue was removed and the thoracic aorta was modified into vascular ring with a diameter of about 2mm, and the vascular ring was carefully mounted up in isolated bath trough at a constant temperature of 37°C. Oxygen was blown to the trough, to which tension transducer and multichannel physiologic recorder were connected. Basal tension was 2g, the vascular ring was equilibrated for 45min$^{-1}$h and the Kreb's solution was replaced at intervals of 15min. After equilibrated, the vascular ring was pre-treated with a potassium chloride solution for 20 min, and eluted. After 15min equilibration, the vascular ring was pre-treated with a potassium chloride solution once again to achieve the physiologically extreme value of vasoconstriction. Next, NA was added in light of different gradient levels ($10^{-7}$, $10^{-6}$, $10^{-5}$, $10^{-4}$mol/L) to observe the vasoconstriction. When reaching the peak value, the value was stabilized in the plateau phase. Then, ACH was added in light of different gradient levels ($10^{-5}$, $10^{-4}$, $10^{-3}$, $10^{-2}$mol/L) to observe vasodilation. During the process of adding NA and ACH, the Kreb's solution cannot be replaced.

In the hypoxia model group, supply of oxygen had been suspended for 20min after pre-treated twice by a potassium chloride solution. Meanwhile, the salvianolic acid L lyophilized powder or Kreb's solution in equal amount was added to bathe together, and followed by addition of NA and ACH in light of different gradient levels. The Kreb's solution cannot be replaced from the starting of hypoxia to the end of the addition of the final concentration gradient of ACH.

The results were statistically analyzed by using *t*-test.

Experimental results

1. Effect on vasoconstriction

As shown in the results, although the salvianolic acid L lyophilized powder had no significant effect on vascular constriction compared with the normal group under the present experimental condition, there was an obviously right shift of the vascular-tension curve. Data were seen in Table 7.

Table 7 Vascular ring constriction data

| Group | dosage (mg/ml) | NA(mol/L) | | | |
|---|---|---|---|---|---|
| | | $10^{-7}$ | $10^{-6}$ | $10^{-5}$ | $10^{-4}$ |
| Normal group | | 0.13±0.22 | 0.53±0.49 | 1.02±0.59 | 1.27±0.62 |
| Hypoxia model group | | -0.01±0.05 | 0.23±0.41 | 1.12±0.31 | 1.37±0.31 |
| hypoxia+ salvianolic acid group | 0.1 | -0.02±0.06 | 0.30±0.33 | 0.84±0.38 | 1.05±0.48 |

*: compared with the normal group, there was a significant difference (P<0.05), #: compared with the model group, there was a significant difference (P<0.05). Effect of the salvianolic acid L lyophilized powder on vascular constriction was seen in Fig. 11.

2. Effect on vasodilation

As shown in this results, compared with the normal group, vasodilation was obviously weakened (P<0.01) at 4 ACH gradient levels in the hypoxia model group under the present experimental condition, while there was no significant difference in the salvianolic acid L group and the normal group. Compared with the hypoxia model group, vasodilation was obviously strengthened (P<0.05) at 3 ACH gradient levels in the salvianolic acid L group. This suggested that the salvianolic acid L group could significantly improve the dysfunction of hypoxia-induced vasodilation. Data were seen in Table 8.

Table 8 Vascular ring dilation data

| Group | dosage (mg/ml) | ACH (mol/L) | | | |
|---|---|---|---|---|---|
| | | $10^{-5}$ | $10^{-4}$ | $10^{-3}$ | $10^{-2}$ |
| Normal group | | -0.09±0.13 | -0.71±0.47 | -0.92±0.51 | -1.09±0.49 |
| Hypoxia model group | | 0.06±0.07** | 0.04±0.07** | -0.03±0.09# | -0.30±0.37*** |
| hypoxia+ salvianolic acid group | 0.1 | -0.09±0.18# | -0.33±0.50# | 0.47±0.60** | -0.67±0.69 |

**: compared with the normal group, there was a significant difference (P<0.01);
***: compared with the normal group, there was a significant difference (P<0.001);
#: compared with the model group, there was a significant difference (P<0.05).

Effect of the salvianolic acid L lyophilized powder on vascular dilation was seen in Fig. 12.

Experimental conclusions:

The salvianolic acid L lyophilized powder had effect on causing the right shift of the vasoconstriction curve of NA to some extent, but no significant difference had been observed. Hypoxia for 20min might lead to a significant decline in gradient dilation of vascular ring caused by ACH in the model group (P<0.01), resulting in the appearance of diastolic dysfunction. On the contrary, the salvianolic acid L [lyophilized powder showed significant enhancement in dilation of anoxic vascular ring at three gradient levels of ACH($10^{-5}$, $10^{-4}$, $10^{-3}$mol/L) (P<0.05). It was confirmed that the salvianolic acid L had a significantly improving effect on vasodilation dysfunction caused by hypoxia.

Discussion for attentions:

1. When preparing the Kreb's solution, calcium chloride and glucose were not added until total addition of other substances in order to prevent turbidity. The Kreb's solution cannot be kept at room temperature for a long time, avoiding flocculent precipitation. Finally, the Kreb's solution was prepared when needed.

2. The cardiac-aorta was taken out in ice bath as far as possible; and the injury to vascular ring caused by apparatuses should be reduced; the cardiac-aorta should be taken out closely enough to vascular arch for the purpose of preventing reduction of vascular activity.

3. Oxygen was exhausted in the form of small bubble which should be as small as possible, oversize bubbles might influence the tension transducer, resulting in the distortion of data.

**Pharmacodynamic example 4** Protective effect of the salvianolic acid L lyophilized powder and the extract thereof on *in vitro* nerve cells

Experimental materials

1. Main apparatus: super-clean bench was produced by Antai Cleaning Equipment Inc; constant temperature $CO_2$ incubator was purchased from Heraeus, Germany; ELISA reader was purchased from BIO-RAD Inc, USA; flat shaking-table was purchased from Jiangsu Guangming Experimental Apparatus Manufacturer and inverted biological microscope was purchased from OLYMPUS, Japan.

2. Main reagents: DMEM high-glucose medium and DMEM glucose-free medium were prepared by GIBCO, trypsin was purchased from SIGMA, fetal bovine serum was purchased from PAA, MTT and DMSO were purchased from SIGMA and LDH test kit was purchased from Nanjing Jiangcheng Bioengineering Institute.

3. Disposable materials: 96-well cell culture microplate was prepared by CORNING.

4. Cell strain: PC12.

Experimental methods:

1. MTT method

a. MTT was added into 96-well microplate with 20μl in each well, and reacted for 4 hours in incubator.

b. The supernatant was discarded, followed by addition of 150μl DMSO in each well and shaken on flat shaking-table for 10min.

c. Absorbance of each well was measured by ELISA reader at wavelength of 570nm to calculate cell survival rate. Cell survival rate %=(OD value of drug administration group/OD value of negative control group) ×100%

2. Determination of LDH activity

The experiment of determination was carried out according to the Specification of LDH test kit provided by Nanjing Jiangcheng Bioengineering Institute. Detailed steps were presented in Table 9.

Table 9 Detailed steps for determination of LDH activity

| | blank (B) | Standard solution (S) | sample tested (U) | Control sample (C) |
|---|---|---|---|---|
| ultrapure water (μl) | 2.5+5 | 2.5 | | 2.5 |
| Standard solution (μl) | | 5 | | |
| Sample (μl) | | | 5 | 5 |
| Matrix fluid (μl) | 12.5 | 12.5 | 12.5 | 12.5 |
| coenzyme (μl) | | | 2.5 | |
| Mixed well, incubated at 37°C for15min | | | | |
| 2,4-dinitrophenylhydrazine | 12.5 | 12.5 | 12.5 | 12.5 |
| Mixed well, incubated at 37°C for15min | | | | |
| 0.4mM NaOH | 125 | 125 | 125 | 125 |

(continued)

| |
|---|
| Mixed well, incubated at 37°C for15min |
| Incubated at room temperature for 3-4min with absorbance detected at 440nm |

LDH activity (U/L)=$(OD_U-OD_C)/(OD_S-OD_B)\times C_S\times N\times 1000$

Wherein, $OD_U$ represented the absorbance of the tested sample, $OD_C$ represented the absorbance of control sample, $OD_B$ represented the absorbance of blank, $OD_S$ represented the absorbance of standard solution, $C_S$ represented the standard concentration of 2mmol/L, N represented the dilution multiple of sample before determination.

Experimental results:

1. Establishment of hydrogen peroxide-damaged model

a. PC12 cells at exponential growth phase in good condition were washed with PBS twice, followed by addition of 0.25% trypsin digestive solution to perform digestion at 37°C for about 1 min. This reaction was ended by addition of blood serum-contained culture medium, centrifuged and resuspended, then counted the cells to prepare a suspension with a cell density of $2\times10^4$-$4\times10^4$ cells/ml.
b. The resulting cell suspension was inoculated into 96-well microplate with 180μl in each well (n=3) and incubated in constant temperature $CO_2$ incubator at 37°C for 24 hours.
c. Grouping and treatments: there were 4 groups: blank control group (PBS), solvent control group (DMSO), model group ($H_2O_2$) and positive control group (Edaravone).
Blank control group: only addition of PBS.
Solvent control group: addition of 0.1% DMSO.
Model group: a concentration of hydrogen peroxide ($H_2O_2$) was respectively at 0.25mM, 0.5 mM and 1mM, a reaction time was 1 hour.
Positive control group: Edaravone (2μg/ml) is added as a positive drug and then pre-treated for 6 hours, into which 0.5mM hydrogen peroxide was added to damage for 1 hour and replaced with freshly-prepared DMEM+10%FBS culture medium, 200μ l per well.
d. The cell activity was determined by MTT method.

Table 10 Establishment of hydrogen peroxide-damaged model

| Group | Final concentration | Survival rate (%) |
|---|---|---|
| Blank control group | 0 | 100±5.65 |
| Solvent control group | 0.1 % DMSO | 93.45±5.21 |
| Model group | 0.25mM $H_2O_2$ | 84.83±7.65 |
| | 0.5mM $H_2O_2$ | 40.31 ±4.63## |
| | 1 mM $H_2O_2$ | 34.36±6.15 ## |
| Positive control group | 2μg/ml (Edaravone) | 53.54±3,66 * |
| *: compared with the model group of 0.5mM $H_2O_2$ (P<0.05), ##: compared with the solvent control group (P<0.01). | | |

As shown in Table 10, PC12 cells survival rate was 40% and inhibition rate was 60% after being treated with 0.5mM $H_2O_2$ for 1 hour. The hydrogen peroxide-damaged model was PC12 cells treated with 0.5mM $H_2O_2$ for 1 hour.

2. Establishment of oxygen-glucose deprivation (OGD) model

a. PC12 cells at exponential growth phase in good condition were washed with PBS twice, followed by addition of 0.25% trypsin digestive solution to perform digestion at 37 °C for about 1 min. This reaction was ended by addition of blood serum-contained culture medium, centrifuged and resuspended, then counted the cells to prepare a suspension with a cell density of $2\times10^4$-$4\times10^4$ cells/ml.
b. The resulting cell suspension was inoculated into 96-well microplate with 180μl in each well (n=3) and incubated in

constant temperature $CO_2$ incubator at 37°C for 24 hours.

c. Grouping and treatments: there were 3 groups: blank control group (normoxia+0.1%DMSO), model group (OGD+0.1%DMSO, oxygen-glucose deprivation) and positive control group (Edaravone).

Model group: the cell in culture microplate was cultured with glucose-free DMEM medium, which was placed in a hypoxic chamber, started to record time for 0.5 hour when $O_2$% was less than 2.6, and then transferred to a routine incubator. The determination was carried out after a period of time of incubation.

Positive control group: Edaravone (2μg/ml) was used as a positive drug. Drug was added and pre-treated for 6 hours, and then replaced with glucose-free medium, 180 μl per well. The drug was added again, placed in a hypoxic chamber, started to record time for 0.5 hour when $O_2$% was less than 2.6, and then transferred to a routine incubator. The determination was carried out after a period of time of incubation.

d. The cell activity was determined by MTT method.

Table 11 Establishment of oxygen-glucose deprivation model

| Group | Final concentration | Survival rate (%) |
|---|---|---|
| Blank control group | 0.1% DMSO | 100±6.66 |
| Model group | 0.1 % DMSO | 42,59±3.06## |
| Positive control group | 2μg/ml (Edaravone) | 48.50±1.81* |
| *: P<0.05, compared with the model group; ##: P<0.01, compared with the blank control group. | | |

As shown in Table 11, the survival rate of oxygen-glucose deprivation-damaged PC12 cells was just 42% and the inhibition rate was 58%. Thus, the oxygen-glucose deprivation model in this study was: the glucose-free DMEM medium had been chosen to culture cells, placed in a hypoxic chamber, started to record time for 0.5 hour when $O_2$% was less than 2.6, and then transferred to a routine incubator. The determination was carried out after a period of time of incubation.

3. Effect of drug on cell survival rate of $H_2O_2$-damaged PC12 cell

a. PC12 cells at exponential growth phase in good condition were washed with PBS twice, followed by addition of 0.25% trypsin digestive solution to perform digestion at 37 °C for about 1 min. This reaction was ended by addition of blood serum-contained culture medium, centrifuged and resuspended, then counted the cells to prepare a suspension with a cell density of $2\times10^4$-$4\times10^4$ cells/ml.

b. The resulting cell suspension was inoculated into 96-well microplate with 180μl in each well (n=3) and incubated in constant temperature $CO_2$ incubator at 37°C for 24 hours.

c. Grouping and treatments: there were 5 groups: blank control group (PBS), solvent control group (DMSO or ethyl acetate), model group ($H_2O_2$), positive control group (Edaravone) and drug treatment group.

Model group: 0.5mM hydrogen peroxide ($H_2O_2$) was used to treat for 1 hour.

Positive control group: Edaravone (2μg/ml), used as a positive drug, was added to cell and pre-treated for 6 hours, into which 0.5mM hydrogen peroxide was added to damage for 1 hour and replaced with freshly-prepared DMEM+10%FBS culture medium.

Drug treatment group: after the cell was inoculated into culture microplate, different tested drugs at different concentration were added firstly and pre-treated for 6 hours with 20μl per well, into which 0.5mM $H_2O_2$ was added to damage for 1 hour, and then replaced with freshly-prepared DMEM+10%FBS culture medium.

d. The supernatant was collected, 20μl per well, for determination of LDH activity.

e. The activity of cell in cell microplate was determined by MTT method.

Table 12 Effect of drug on cell survival rate of $H_2O_2$-damaged PC12 cell

| Group | Final concentration | Survival rate (%) |
|---|---|---|
| Solvent control group (DMSO) | 0.1 % DMSO | 100±0.66 |
| | 0.01 % DMSO | 100±0.48 |
| | 0.001 % DMSO | 100±0.4 |

(continued)

| Group | Final concentration | Survival rate (%) |
|---|---|---|
| Solvent control group (EtOAc) | 0.1 % EtOAc | 100±0.86 |
| | 0.01 % EtOAc | 100±1.38 |
| | 0.001% EtOAc | 100±0.96 |
| Model group (0.5mM $H_2O_2$+DMSO) | 0.5mM $H_2O_2$+0.1 % DMSO | 47.32±1.15## |
| | 0.5mM $H_2O_2$+0.01 % DMSO | 43.65±3.0 ## |
| | 0.5mM $H_2O_2$+0.001 % DMSO | 40.67±3.61 ## |
| Model group (0.5mM $H_2O_2$+ EtOAc) | 0.5mM $H_2O_2$+0.1 % EtOAc | 38.45±2.15 ## |
| | 0.5mM $H_2O_2$+0.01 % EtOAc | 39.28±1.75 ## |
| | 0.5mM $H_2O_2$+0.001 % EtOAc | 39.65±1.84 ## |
| Positive control group (Edaravone) | 2μg/ml | 58.18t2.$B_4$ ** |
| | 0.2μg/ml | 44.2±6.11 |
| | 0.02μg/ml | 47.6±2 * |
| Drug treatment group (No.1 extract of the salvianolic acid L) | 2μg/ml | 50.65±5.65 |
| | 0.2μg/ml | 44.75±4.56 |
| | 0.02μg/ml | 43.2±1.6 |
| Drug treatment group (No.2 extract of the salvianolic acid L) | 2μg/ml | 40.09±2.39 |
| | 0.2μg/ml | 43.23±5.72 |
| | 0.02μg/ml | 44.67±6.42 |
| Drug treatment group (yellowish powder: the salvianolic acid L) | 2μg/ml | 48.34±0.25 |
| | 0.2μg/ml | 44.89±0.48 |
| | 0.02μg/ml | 47.2±0.8 * |
| *: P<0.05, compared with the model group (0.5mM $H_2O_2$+DMSO);<br>**: P<0.01, compared with the model group (0.5mM $H_2O_2$+DMSO);<br>##: P<0.01, compared with the solvent control group (DMSO). | | |

Drug concentrations were respectively prepared with DMSO at 0.1%, 0.01% and 0.001%, which should be compared with the solvent control group having corresponding concentration. Wherein the drug treatment group was compared with the model group (0.5mM $H_2O_2$+EtOAc), while the model group ($H_2O_2$+EtOAc) was compared with the solvent control group (EtOAc).

Table 13 Effect of drug on LDH activity of $H_2O_2$-damaged PC12 cell

| Group | Final concentration | LDH activity (U/L) |
|---|---|---|
| Solvent control group (DMSO) | 0.1 % DMSO | 325.71 ±11.42 |
| | 0.01 % DMSO | 348.5±16.33 |
| | 0.001 % DMSO | 374.16±3.81 |
| Solvent control group (EtOAc) | 0.1 % EtOAc | 313.9±16.33 |
| | 0.01% EtOAc | 329.97±16.34 |
| | 0.001% EtOAc | 342.29±30.13 |

(continued)

| Group | Final concentration | LDH activity (U/L) |
|---|---|---|
| Model group (0.5mM $H_2O_2$+DMSO) | 0.5mM $H_2O_2$+0.1 % DMSO | 608.5±16.33 ## |
| | 0.5mM $H_2O_2$+0.01 % DMSO | 599.55±0 ## |
| | 0.5mM $H_2O_2$+0.001 % DMSO | 617.45±16.33 ## |
| Model group (0.5mM $H_2O_2$+ EtOAc) | 0.5mM $H_2O_2$+0.1 % EtOAc | 596.95±33.08 ## |
| | 0.5mM $H_2O_2$+0.01 % EtOAc | 590.74±31.43 ## |
| | 0.5mM $H_2O_2$+0.001 % EtOAc | 610.81 ±11.55 ## |
| Positive control group (Edaravone) | 2μg/ml | 523.49±5.17 ** |
| | 0.2μg/ml | 568.23±11.55 ** |
| | 0.02μg/ml | 532.44±11.55 ** |
| Drug treatment group (No.1 extract of the alvianolic acid L) | 2μg/ml | 465.32±32.68 * |
| | 0.2μg/ml | 416.11±63.91 ** |
| | 0.02μg/ml | 483.22±21.92 ** |
| Drug treatment group (No.2 extract of the salvianolic acid L) | 2μg/ml | 4.87,70±11.55 ** |
| | 0.2μg/ml | 407.16±34.66 ** |
| | 0.02μg/ml | 559.28±27.82 |
| Drug treatment group (yellowish powder: the salvianolic acid L) | 2μg/ml | 487.70±72.51 |
| | 0.2μg/ml | 474.27±71.96 * |
| | 0.02μg/ml | 550.336±25.83 |
| *: P<0.05, compared with the model group (0.5mM $H_2O_2$+DMSO);<br>**: P<0.01, compared with the model group (0.5mM $H_2O_2$+DMSO);<br>##: P<0.01 , compared with the solvent control group (DMSO). | | |

Wherein the drug treatment group was compared with the model group (0.5mM $H_2O_2$+EtOAc), while the model group ($H_2O_2$+EtOAc) was compared with the solvent control group (EtOAc).

4. Effect of drug on the survival rate of PC12 cell of oxygen-glucose deprivation

a. PC12 cells at exponential growth phase in good condition were washed with PBS twice, followed by addition of 0.25% trypsin digestive solution to perform digestion at 37 °C for about 1 min. This reaction was ended by addition of blood serum-contained culture medium, centrifuged and resuspended, then counted the cells to prepare a suspension with a cell density of $2\times10^4$-$4\times10^4$ cells/ml.

b. The resulting cell suspension was inoculated into 96-well microplate with 180μl in each well (n=3) and incubated in constant temperature $CO_2$ incubator at 37°C for 24 hours.

c. Grouping and treatments: there were 4 groups: blank control group (normoxia+0.1 %DMSO), model group (OGD+DMSO, oxygen-glucose deprivation), positive control group (Edaravone) and the drug treatment group.

Model group: the culture medium for the cells in microplate was changed to glucose-free DMEM, placed in a hypoxic chamber, and started to record time for 0.5 hour when $O_2$% was less than 2.6, and then transferred to a routine incubator to culture overnight.

Positive control group: Edaravone (2μg/ml) was used as a positive drug. Drug was added and pre-treated for 6 hours, and then the culture medium was replaced with glucose-free DMEM medium, 180μl per well. The drug was added again, placed in a hypoxic chamber, started to record time for 0.5 hour when $O_2$% was less than 2.6, and then transferred to a routine incubator to culture overnight.

Drug treatment group: the drug at different concentrations was added and pre-treated for 6 hours, the culture medium was changed to glucose-free DMEM medium, 180μl per well. The drug was added again, placed in a hypoxic chamber,

started to record time for 0.5 hour when $O_2$% was less than 2.6, and then transferred to a routine incubator to culture overnight.

d. Resulting supernatant was collected the next day with 20μl per well, which was used for determination of LDH activity.

e. The activity of cell was determined by MTT method.

Table 14 Effect of drug on the cell survival rate of PC12 cell of oxygen-glucose deprivation

| Group | Final concentration | Survival rate (%) |
|---|---|---|
| Blank control group (Normoxia+DMSO) | 0.1 % DMSO | 100±0.27 |
| | 0.01 % DMSO | 100±0.68 |
| | 0.001 % DMSO | 100±0.77 |
| Blank control group (Normoxia+EtOAc) | 0.1 % EtOAc | 100±1.07 |
| | 0.01 % EtOAc | 100±0.45 |
| | 0.001 % EtOAc | 100±1.02 |
| Model group (OGD+ EtOAc) | 0.1 % EtOAc | 29.19±1.65 ## |
| | 0.01 % EtOAc | 30.15±1.47 ## |
| | 0.001 % EtOAc | 31.26±2.08 ## |
| Model group (OGD+DMSO) | 0.1 % DMSO | 26.65±1.17 ## |
| | 0.01 % DMSO | 31.75±0.77 ## |
| | 0.001 % DMSO | 38.54±1.75 ## |
| Positive control group (OGD+Edaravone) | 2μg/ml | 39.99±0.55 ** |
| | 0.2μg/ml | 36.37±2.52* |
| | 0.02μg/ml | 44.75±1.02 ** |
| Treatment group (No.1 extract of the salvianolic acid L) | 2μg/ml | 42.35±1.75 ** |
| | 0.2μg/ml | 40.13±0.34 ** |
| | 0.02μg/ml | 34.33±0.9 |
| Treatment group (No.2 extract of the salvianolic acid L) | 2μg/ml | 40.58±2.79 * |
| | 0.2μg/ml | 46.6±2.42 ** |
| | 0.02μg/ml | 46.83±1.5 ** |
| Treatment group (yellowish powder: the salvianolic acid L) | 2μg/ml | 35.96±1.44 |
| | 0.2μg/ml | 36.68±2.72 * |
| | 0.02μg/ml | 47.74±1.72 ** |
| *: P<0.05, compared with the model group (OGD+DMSO); **: P<0.01, compared with the model group (OGD+DMSO); ##: P<0.01, compared with the blank control group (Normoxia+DMSO). Wherein, the drug treatment group was compared with the blank control group (OGD+EtOAc), while the model group (OGD+EtOAc) was compared with the blank control group (Normoxia+DMSO). | | |

f. LDH activity

Table 15 Effect of drug on LDH activity of PC12 cell of oxygen-glucose deprivation (OGD)

| Group | Final concentration | LDH activity (U/L) |
|---|---|---|
| Blank control group (Normoxia+DMSO) | 0.1 % DMSO | 21.08±5.17 |
| | 0.01 % DMSO | 24.01±7.31 |
| | 0.001 % DMSO | 22.01±11.55 |
| Blank control group (Normoxia+EtOAc) | 0.1 % EtOAc | 23.96±10.33 |
| | 0.01 % EtOAc | 20.78±5.17 |
| | 0.001 % EtOAc | 22.37±7.31 |
| Model group (OGD+ EtOAc) | 0.1 % EtOAc | 46.35+11.55 ## |
| | 0.01 % EtOAc | 42.39±5.17 ## |
| | 0.001 % EtOAc | 58.92±10.33 ## |
| Model group (OGD+DMSO) | 0.1 % DMSO | 49.22±5.17 ## |
| | 0.01 % DMSO | 40.27± 5.17 ## |
| | 0.001 % DMSO | 62.64±14.61 ## |
| Positive control (OGD+Edaravone) group | 2μg/ml | 31.32±5.17 ** |
| | 0.2μg/ml | 22.37±5.17 ** |
| | 0.02μg/ml | 40.27±5.17 * |
| Drug treatment group (No.1 extract of the salvianolic acid L) | 2μg/ml | 31.32±5.17 ** |
| | 0.2μg/ml | 44.74±10.33 |
| | 0.02μg/ml | 80.54±30.13 |
| Drug treatment group (No.2 extract of the salvianolic acid L) | 2μg/ml | 102.91±25.83 |
| | 0.2μg/ml | 31.32±5.17 * |
| | 0.02μg/ml | 67.11±5.17 |
| Drug treatment group (yellowish powder: the salvianolic acid L) | 2μg/ml | 40.27±5.17 * |
| | 0.2μg/ml | 31.32±11.55 |
| | 0.02μg/ml | 53.69±29.23 |
| *: P<0.05, compared with the model group (OGD+DMSO); **: P<0.01, compared with the model group (OGD+DMSO); ##: P<0.01, compared with the blank control group (Normoxia+ DMSO). Wherein, the drug treatment group was compared with the blank control group (OGD+EtOAc), while the model group (OGD+EtOAc) was compared with the blank control group (Normoxia+EtOAc). | | |

Conclusions:

Results of the experiment: When treated with the salvianolic acid L lyophilized powder at the dosage of 0.02μg/ml, the cell survival rate of $H_2O_2$-damaged PC12 cells was 47% (P<0.05); while at the dosage of 0.2μg/ml, LDH activity was 474 (P<0.05). As for No.1 extract of the salvianolic acid L at the dosage of 0.02, 0.2 and 2μg/ml, LDH activities were 483(P<0.01), 416 (P<0.01) and 465 (P<0.05) respectively; while for No.2 extract of the salvianolic acid L at the dosage of 0.2 and 2μg/ml, LDH activities were 407 (P<0.01) and 488 (P<0.01) respectively, compared with the model group, they both had an effect of reducing LDH activity. When treated with the salvianolic acid L lyophilized powder at the dosage of 0.02 and 0.2μg/ml, the survival rates of OGD cells were 48% (P<0.01) and 37% (P<0.05) respectively. With regard to No.1 extract of the salvianolic acid L at the dosage of 0.2 and 2μg/ml, the survival rates were 40% (P<0.01) and 42% (P<0.01) respectively, while about No.2 extract of the salvianolic acid L at the dosage of 0.02, 0.2 and 2μg/ml, the survival rates were 47% (P<0.01), 47% (P<0.01) and 41% (P<0.05) respectively. When treated with the salvianolic acid L lyophilized powder at the dosage of 2μg/ml, LDH activity was 40 (P<0.05). Moreover, with regard to No.1 extract of the salvianolic acid L at the dosage of 2μg/ml, LDH activity was 31 (P<0.01), while No.2 extract of the salvianolic acid

L at the dosage of 0.2μg/ml, LDH activity was 31 ($P<0.05$).

As shown in the experiment, not only did the salvianolic acid L lyophilized powder have a significantly improving effect on *in vitro* neuronal injury caused by OGD or $H_2O_2$, but also increased survival rate of the cells. Therefore, it was confirmed that the salvianolic acid L had functions of protecting nerve cells in condition of oxygen deprivation, glucose deprivation and over-oxidation.

Pharmacodynamic example 5 Protective effect of the salvianolic acid L lyophilized powder and extracts on experimental acute myocardial ischemia in rats

Experimental materials:

1. Test materials and reagents: pituitrin (Pit) injection was produced by Nanjing Xinbai Pharmaceutical Co., Ltd. with the batch No. of 070302. Normal saline was produced by Tianjin Tian'an Pharmaceutical Co., Ltd. with the batch No. of 200605241, specification: 500ml/bottle.

2. Main apparatus: MedLab® 8-chanal biophysiological recorder was produced by Nanjing Medease Science and Technology Co., Ltd.

3. Animals: SD rats, male or female in proper body-weight, were provided by Beijing Vital River Laboratory Animal Technology Co., Ltd., with the certificate No. of SCXK (Jing) 2007-0001. All rats were fed with rat special diet (produced by Beijing Keaoxieli Diet Co., Ltd.) and tap water in animal feeding room at room temperature of 20-25°C, illuminated for 12 hours.

Experimental methods

1. Design of administration dose

Content of No.1 extract was 6.825g crude drugs/g and No.2 extract was 4.162 g crude drugs/g.

For both No.1 and No.2 extracts, there were two groups of high-dose and low-dose: 1.086g crude drugs/kg and 0.543g crude drugs/kg respectively. According to the dose conversion of the crude drugs, administration dose of the salvianolic acid L lyophilized powder in high-dose No.1 extract was 4.57mg/kg, and 2.33mg/kg in low-dose group. No salvianolic acid L was found in No.2 extract.

Administration dose of the salvianolic acid L lyophilized powder was 10.0mg/kg and 5.0mg/kg.

2. Grouping

2.1 Screening of animals

Before formal experiment, rats were injected via vena caudalis with pituitrin (Pit) (1 U/kg). Normal ECG and the ECG of 5min after injection were recorded to observe J point elevation and T wave abnormality. Animals who had abnormal ECG before injection or who were insensitive to Pit were rejected.

2.2 Grouping of animals

Desirable rats were divided into 7 groups: ① model control group, ② No.1 extract of Danshen low-dose group (A group), ③ No.1 extract of Danshen high-dose group (B group), ④ No.2 extract of Danshen low-dose group (C group), ⑤ No.2 extract of Danshen high-dose group (D group), ⑥ Salvianolic acid L lyophilized powder low-dose group (E group), and ⑦ Salvianolic acid L lyophilized powder high-dose group (F group).

3. Experimental methods

SD rats, half male and half female, were randomly divided into groups, 8 animals in each group. The rats in the treatment groups were drenched with aqueous suspensions of different sample each day, while the rats in the model control group were drenched with equal volume of normal saline. All animals were consecutively administered for 7 days. 40min after final administration, the rats were anesthetized and connected with devices to record lead II normal ECG. The pituitrin (Pit) was injected at a constant speed in the dosage of 1 U/kg body weight via vena caudalis within

about 10s. ECG changes were recorded at 0s, 5s, 10s, 15s, 30s, 45s, 1 min, 2min, 3min, 4min, 5min, 10min and 15min after administration. Differences between pre injection and post injection of Pit of each group as well as between the treatment group and the model control group were compared to analyze changes of J point and T wave, and the data were analyzed by *t*-test.

Experimental results

1. Effect on J point

As shown in the results, compared with the model control group, the elevation extent of J point of ECG in F group (Salvianolic acid L lyophilized powder high-dose group) is less at 15s, 30s and 45s in pituitrin-caused acute myocardial ischemia and the difference had statistical significance under the present experimental condition (P<0.05). Compared with the model control group, the elevation extent of J point of ECG in B group (No.1 extract of Danshen high-dose group) is less at 15s and the difference had statistical significance (P<0.05). Compared with the model control group, however, other groups showed no significant difference at each time-point. Data were seen in Table 16.

Table 16 Effect of the different extracts administration groups on ECG J point in acute myocardial ischemia

| Groups | Time points | | | | | | | | | | |
|---|---|---|---|---|---|---|---|---|---|---|---|
| | normal | 0s | 15s | 30s | 45s | 1 min | 2min | 3min | 4min | 5min | 10min | 15min |
| Model | -0.059 ±0.083 | -0.029 ±0.070 | 0,020 ±0.059 | -0.028 ±0.070 | -0.031 ±0.100 | -0.004 ±0.055 | -0.040 ±0.059 | -0.114 ±0.079 | -0.132 ±0.070 | -0.072 ±0.061 | -0.033 ±0.043 | -0.040 ±0.075 |
| A group | -0.075 ±0.096 | 0.039 ±0.074 | 0.046 ±0.060 | 0.008 ±0.073 | -0.009 ±0.099 | 0.004 ±0.090 | 0.002 ±0.098 | 0.005 ±0.079* | 0.013 ±0.087* | -0.003 ±0.082 | -0.0006 ±0.102 | 0.014 ±0.119 |
| B group | -0.045 ±0.061 | -0.051 ±0.077 | -0.067 ±0.093* | -0.041 ±0.077 | -0.018 ±0.072 | 0.013 ±0.076 | -0.036 ±0.074 | -0.040 ±0.054 | -0.042 ±0.060* | -0.033 ±0.066 | -0.086 ±0.117 | -0.085 ±0.102 |
| C group | -0.036 ±0.064 | -0.060 ±0.087 | -0.020 ±0.096 | -0.057 ±0.112 | -0.020 ±0.101 | -0.027 ±0.084 | -0.016 ±0.052 | 0.001 ±0.054* | -0.021 ±0.077* | -0.030 ±0.090 | -0.005 ±0.109 | -0.020 ±0.106 |
| D group | 0.003 ±0.076 | 0.069 ±0.070* | 0.060 ±0.075 | 0.017 ±0.098 | 0.080 ±0.077* | 0.106 ±0.070* | 0.035 ±0.133 | -0.020 ±0.223 | 0.077 ±0.041* | 0.069 ±0.032* | 0.088 ±0.085* | 0.027 ±0.111 |
| E group | -0.032 ±0.046 | -0.040 ±0.057 | -0.023 ±0.076 | -0.051 ±0.092 | -0.030 ±0.081 | -0.026 ±0.074 | -0.019 ±0.062 | 0.003 ±0.079 | -0.018 ±0.107 | -0.023 ±0.087 | -0.015 ±0.108 | -0.018 ±0.098 |
| F group | -0.024 ±0.056 | -0.038 ±0.047 | -0.070 ±0.045* | -0.068 ±0.028* | -0.049 ±0.010* | -0.006 ±0.087 | -0.032 ±0.153 | -0.087 ±0.093 | -0.079 ±0.076 | -0.069 ±0.081 | -0.088 ±0.088 | -0.027 ±0.120 |

*: Compared with the model control group, there was a significant difference (P<0.05)

2. Effect on T wave

As shown in the results, compared with the model control group, the elevation extent of T wave of ECG of F group (Salvianolic acid L lyophilized powder high-dose group) at 15s and 30s is less, and the difference had statistical significance under the present experimental condition ($P<0.05$). Similarly, compared with the model control group, the elevation extent of T wave of ECG in B group (No.1 extract of Danshen high-dose group) at 15s is less, and the difference had statistical significance ($P<0.05$). Compared with the model control group, however, other groups showed no significant difference at each time-point. Data were seen in Table 17.

Table 17 Effect of the different extracts administration groups on ECG T wave in acute myocardial ischemia

| Groups | Time points | | | | | | | | | | |
|---|---|---|---|---|---|---|---|---|---|---|---|
| | normal | 0s | 15s | 30s | 45s | 1min | 2min | 3min | 4min | 5min | 10min | 15min |
| Model | 0.095 ±0.092 | 0.150 ±0.078 | 0.260 ±0.082 | 0.120 ±0.099 | 0.089 ±0.118 | 0.161 ±0.016 | 0.120 ±0.105 | 0.043 ±0.161 | 0.057 ±0.196 | 0.124 ±0.158 | 0.151 ±0.097 | 0.121 ±0.160 |
| A group | 0.163 ±0.091 | 0.416 ±0.368 | 0.247 ±0.072 | 0.199 ±0.069 | 0.170 ±0,116 | 0.202 ±0.063 | 0.208 ±0.086 | 0.222 ±0.053* | 0.160 ±0.100 | 0.208 ±0.064 | 0.164 ±0.146 | 0.135 ±0.176 |
| B group | 0.156 ±0.090 | 0.171 ±0.143 | 0.165 ±0.064* | 0.104 ±0.163 | 0.121 ±0.117 | 0.148 ±0.065 | 0.167 ±0.053 | 0.151 ±0.065 | 0.105 ±0.082 | 0.161 ±0.075 | 0.114 ±0.132 | 0.075 ±0.160 |
| C group | 0.122 ±0.131 | 0.121 ±0.167 | 0.257 ±0.246 | 0.229 ±0.098 | 0.244 ±0.073* | 0.247 ±0.086* | 0.226 ±0.085* | 0.188 ±0.066* | 0.176 ±0.141 | 0.164 ±0.143 | 0.177 ±0.063 | 0.170 ±0.129 |
| D group | 0.177 ±0.101 | 0.258 ±0.079* | 0.287 ±0.076 | 0.249 ±0.068* | 0.354 ±0.222* | 0.290 ±0.105* | 0.192 ±0.213 | 0.126 ±0.279 | 0.235 ±0.043 | 0.233 ±0.033 | 0.205 ±0.070 | 0.189 ±0.185 |
| E group | 0.102 ±0.103 | 0.130 ±0.201 | 0.207 ±0.136 | 0.194 ±0.159 | 0.187 ±0.173 | 0.139 ±0.186 | 0.142 ±0.185 | 0.121 ±0.166 | 0.116 ±0.119 | 0.204 ±0.243 | 0.197 ±0.163 | 0.181 ±0.121 |
| F group | 0.097 ±0.141 | 0.124 ±0.179 | 0.151 ±0.096* | 0.094 ±0.084* | 0.142 ±0.102 | 0.139 ±0.175 | 0.130 ±0.201 | 0.096 ±0.179 | 0.137 ±0.143 | 0.163 ±0.133 | 0.185 ±0.107 | 0.195 ±0.235 |

*: Compared with the model control group, there was a significant difference ($P<0.05$)

Conclusions:

Compared with the model control group, the elevation extent of J point of ECG and T wave in F group (Salvianolic acid L lyophilized powder high-dose group) is less at 15s and 30s, and the difference had statistical significance ($P<0.05$).
Compared with the model control group, both J point and T wave at 15s are significantly decreased in B group (No.1 extract of Danshen high-dose group) ($P<0.05$).
Compared with the model control group, other groups showed no significant decrease in J point and T wave at each time-point.
As shown in result, under this study, the salvianolic acid L lyophilized powder (10mg/kg) and No.1 extract containing the salvianolic acid L at concentration of 4.67mg/kg had effect of anti-acute myocardiac ischemia, but no effect of anti-acute myocardiac ischemia had been observed under the experimental dosage in No.2 extract that did not contain the salvianolic acid L.

Discussions for attentions:

1. Definition of J point: the combination point of the ending of QRS wave group and ST segment.

2. Due to the constrictive effect on coronary vessel of pituitrin, intravenous injection of pituitrin can induce acute myocardial ischemia in normal rats, resulting in obvious elevation of both J point and T wave in ECG. The shift of J point in the drug treatment group had significantly recovered and T wave had decreased to normal level gradually after the tested drug was administered, this suggested that the drug had antagonistic effect on acute myocardial ischemia induced by the constrictive effect of pituitrin on coronary vessel. The drug, having therapeutic effect whether on I phase abnormality (induced by pituitrin within 0-45s) or II phase abnormality (induced by pituitrin within 45s-15min), was usually believed to have the effect of anti-myocardial ischemia.

3. During the experiment, the pituitrin of the same batch number should be used to avoid the effect of the potency unit of drug on the experimental results. Pituitrin should be injected at an interval of more than 2 hours to avoid drug resistance. Preferably, the selected animals are used every other day.

## Claims

**1.** A *trans*-form of salvianolic acid L having the general formula (I), its pharmaceutically-acceptable salts and solvates:

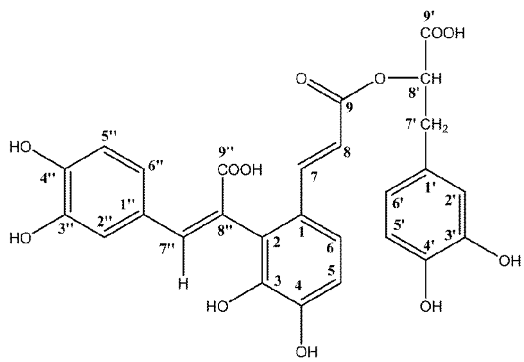

formula (I)

**2.** A method for preparing the salvianolic acid L of claim 1, comprising following steps:

a) extraction: extracting *Radix Salviae Miltiorrhizae* crude drug or a mixture of *Radix Salviae Miltiorrhizae* and other crude drugs with water, adding alcohol to precipitate and obtain a supernatant, then concentrating the supernatant to obtain an extract;
b) separation: dissolving the extract of the step a) in water, applying on a macroporous absorbent resin and

then eluting the resin with water to obtain an eluent, acidifying the eluent, applying the acidified eluent again on the macroporous absorbent resin, washing the resin with an acidic aqueous solution to remove impurities and then eluting the resin with ethanol to obtain an ethanol eluent, concentrating the ethanol eluent to obtain an extract;
c) purification: applying the extract of the step b) on the silica gel column, isocratic eluting with a mobile phase of chloroform, methanol and formic acid; collecting the eluent; monitoring the whole elution process by TLC, combining characteristically analogous eluents to obtain the salvianolic acid L.

**3.** The method according to claim 2, **characterized in that**: in the step a), said *Radix Salviae Miltiorrhizae* crude drug or a mixture of *Radix Salviae Miltiorrhizae* and other crude drugs is sliced into decoction pieces; said water-extraction is as follows:

decocting the crude drug with water of 4-8 times the volume of the crude drug for 1.5-3.5 hours, filtering; decocting drug residue with water of 3-6 times the volume of the drug residue for 1-3 hours, filtering; and combining the filtrate, concentrating the filtrate to obtain an extract with a relative density of 1.11-1.28 (80°C); said alcohol-precipitation is as follows: adding 95% ethanol into the extract to precipitate until the content of the ethanol being 65%-70% and standing still for 12-36 hours, concentrating the supernatant by recovering ethanol under reduced pressure condition, and obtaining an extract with a relative density of 1.30-1.38 (60°C);
In the step b), the final extract of step (a) is applied on macroporous absorbent resin column, the weight ratio of the crude drug to macroporous absorbent resin is 5:1-1:1, the resin column is washed with water of 8-15 times the bed volume to obtain a water eluent, and hydrochloric acid is added into the water eluent to adjust its pH value to 2.2-3.5; said acidic eluent is applied on the macroporous absorbent resin column again with the weight ratio of the crude drug to the macroporous absorbent resin of 5:1-1:1, the column is washed with hydrochloric acid having a pH value of 2.2-3.5 until the eluent being nearly colorless; 50%-95% ethanol of 3-8 times the bed volume is used to wash the column, and the eluent is concentrated to obtain an extract without alcoholic smell; said macroporous absorbent resin is one macroporous absorbent resin selected from the group consisting of AB-8, HPD450, HPD700, D101, D4020 or X5;
In the step c), the extract obtained by concentration in the step b) is dissolved with organic solvent, mixed with chromatographic silica gel, the well-mixed sample is placed on the well-packed silica gel column, the column is eluted with a mobile phase of chloroform : methanol : formic acid with a volume ratio of 90:10:3-40:10:0.5.

**4.** The method according to claim 2, **characterized in that**: in the step a), said water-extraction is as follows: decocting the crude drug with water of 4 times the volume of the crude drug for 2 hours, filtering; decocting drug residue with water of 3 times the volume of the drug residue for 1 hour, filtering; and combining the filtrate, concentrating the filtrate to obtain an extract with a relative density of 1.2; said alcohol-precipitation is as follows: adding 95% ethanol into the extract to precipitate until the content of the ethanol being 70% and standing still for 24 hours, concentrating the supernatant by recovering ethanol under reduced pressure condition, and obtaining an extract with a relative density of 1.37;
In the step b), the final extract of step (a) is applied on a macroporous absorbent resin column, the weight ratio of the crude drug to macroporous absorbent resin is 4:1, the resin column is washed with water of 12 times the bed volume to obtain a water eluent, and hydrochloric acid is added into the water eluent to adjust its pH value to 3.0; said acidic eluent is applied on the macroporous absorbent resin column again with the weight ratio of the crude drug to the macroporous absorbent resin of 4:1, the column is washed with hydrochloric acid having a pH value of 3.0 until the eluent being nearly colorless; 95% ethanol of 4 times the bed volume is used to wash the column, and the eluent is concentrated to obtain an extract without alcoholic smell; said macroporous absorbent resin is AB-8;
In the step c), the extract obtained by concentration in the step b) is dissolved with methanol, mixed with 200-300 mesh of chromatographic silica gel, the well-mixed sample is placed on the well-packed 200-300 mesh silica gel column, the column is eluted with a mobile phase of chloroform : methanol : formic acid with a volume ratio of 50:10:2.

**5.** The method according to any one of claims 2-4, **characterized in that**, an alkali aqueous solution is used in said water-extraction in the step a), said alkali is at least one selected from the group consisting of sodium bicarbonate, sodium carbonate, sodium hydroxide, potassium bicarbonate, potassium carbonate and potassium hydroxide.

**6.** The method according to claim 5, **characterized in that**, said alkali aqueous solution is a sodium bicarbonate aqueous solution or a sodium hydroxide aqueous solution.

**7.** The method according to claim 6, **characterized in that**, said alkali aqueous solution is a sodium bicarbonate aqueous solution in a concentration of 0.30%-0.68% or a sodium hydroxide aqueous solution in a concentration of

0.0025‰-0.004‰.

**8.** The method according to claim 7, **characterized in that**, said alkali aqueous solution is a sodium bicarbonate aqueous solution in a concentration of 0.45%.

**9.** The method according to any one of claims 2-8, **characterized in that**, the step a) further comprises an alcohol-extraction before the water-extraction.

**10.** The method according to claim 9, wherein said alcohol-extraction is as follows: decocting twice with 50-95% ethanol of 5-8 times the volume of the crude drug, 1-2 hours each time, filtering, discarding the ethanol-extraction solution, extracting drug residue with water.

**11.** A pharmaceutical composition comprising said salvianolic acid L of claim 1 and pharmaceutically-acceptable carries.

**12.** A use of said salvianolic acid L of claim 1 in the preparation of a medicament for treating cardiovascular diseases.

**13.** The use according to claim 12, wherein said cardiovascular disease is at least one disease selected from the group consisting of hypoxia-induced vasodilatation dysfunction, *in vitro* neuronal injury caused by oxygen deprivation, glucose deprivation and over-oxidation status, and acute myocardial ischemia.

**14.** A use of said salvianolic acid L of claim 1 in the preparation of a medicament having an activity of scavenging free radical.

**15.** A use of said salvianolic acid L of claim 1 in the preparation of a medicament having an activity of preventive anti-oxidation function.

## Patentansprüche

**1.** *Trans*-Form von Salvianolsäure L mit der allgemeinen Formel (I), ihre pharmazeutisch akzeptablen Salze und Solvate:

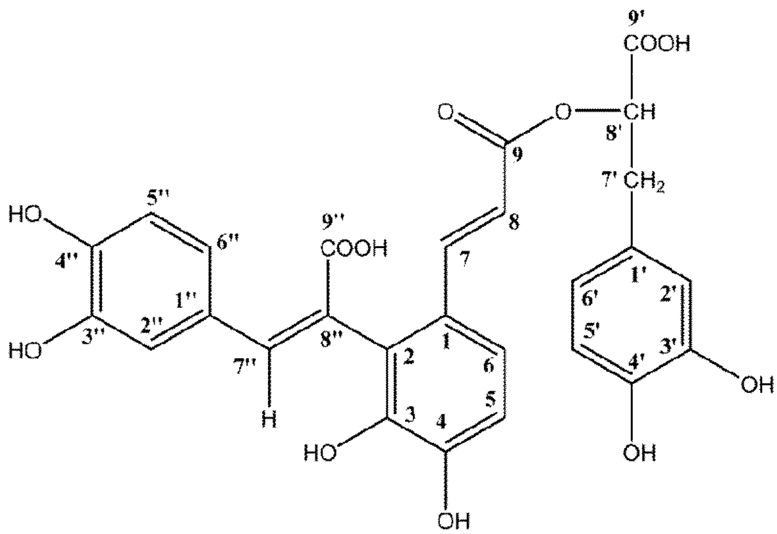

Formel (I)

**2.** Verfahren zur Herstellung der Salvianolsäure L nach Anspruch 1, das folgende Schritte umfasst:

a) Extraktion: Extrahieren von *Radix Salviae Miltiorrhizae*-Rohdroge oder eines Gemischs aus *Radix Salviae Miltiorrhizae* und weiteren Rohdrogen mit Wasser, Zugeben von Alkohol zum Ausfällen und Erhalten eines

Überstands, dann Konzentrieren des Überstands, um einen Extrakt zu erhalten;
b) Trennung: Lösen des Extrakts von Schritt a) in Wasser, Aufbringen auf ein makroporöses absorptionsfähiges Harz und dann Eluieren des Harzes mit Wasser, um ein Elutionsmittel zu erhalten, Ansäuern des Elutionsmittels, erneutes Aufbringen des angesäuerten Elutionsmittels auf das makroporöse absorptionsfähige Harz, Waschen des Harzes mit einer sauren wässrigen Lösung, um Verunreinigungen zu entfernen, und dann Eluieren des Harzes mit Ethanol, um ein Ethanolelutionsmittel zu erhalten, Konzentrieren des Ethanolelutionsmittels, um einen Extrakt zu erhalten;
c) Reinigung: Aufbringen des Extrakts von Schritt b) auf die Kieselgelsäule, isokratisches Eluieren mit einer mobilen Phase aus Chloroform, Methanol und Ameisensäure; Auffangen des Elutionsmittels; Überwachen des gesamten Elutionsverfahrens mit TLC, Kombinieren charakteristisch analoger Elutionsmittel, um die Salvianolsäure L zu erhalten.

**3.** Verfahren nach Anspruch 2, **dadurch gekennzeichnet, dass**:

in Schritt a) die *Radix Salviae Miltiorrhizae*-Rohdroge oder ein Gemisch aus *Radix Salviae Miltiorrhizae* und weiteren Rohdrogen in Auskochstücke geschnitzelt wird; die Wasserextraktion wie folgt abläuft: Auskochen der Rohdroge mit Wasser mit dem 4-8-fachen Volumen der Rohdroge für 1,5-3,5 Stunden, Filtern; Auskochen von Drogenrückstand mit Wasser mit dem 3-6-fachen Volumen des Drogenrückstands für 1-3 Stunden, Filtern; und Kombinieren des Filtrats, Konzentrieren des Filtrats, um einen Extrakt mit einer relativen Dichte von 1,11-1,28 (80 °C) zu erhalten; die Alkoholausfällung wie folgt abläuft: Zugeben von 95-%igem Ethanol zu dem Extrakt zum Ausfällen, bis der Ethanolgehalt 65 %-70 % beträgt, und Stehenlassen für 12-36 Stunden, Konzentrieren des Überstands durch Rückgewinnen von Ethanol bei Unterdruck und Erhalten eines Extrakts mit einer relativen Dichte von 1,30-1,38 (60 °C);
in Schritt b) der endgültige Extrakt von Schritt a) auf eine makroporöse absorptionsfähige Harzsäule aufgebracht wird, das Gewichtsverhältnis der Rohdroge zu makroposösem absorptionsfähigem Harz 5:1-1:1 beträgt, die Harzsäule mit Wasser mit dem 8-15-fachen Bettvolumen gewaschen wird, um ein Wasserelutionsmittel zu erhalten, und
Chlorwasserstoffsäure zu dem Wasserelutionsmittel zugegeben wird, um seinen pH-Wert auf 2,2-3,5 einzustellen; das saure Elutionsmittel mit dem Gewichtsverhältnis der Rohdroge zu dem makroporösen absorptionsfähigen Harz von 5:1-1:1 erneut auf die makroporöse absorptionsfähige Harzsäule aufgebracht wird, die Säule mit Chlorwasserstoffsäure mit einem pH-Wert von 2,2-3,5 gewaschen wird, bis das Elutionsmittel nahezu farblos ist; 50-95-%iges Ethanol mit dem 3-8-fachen Bettvolumen verwendet wird, um die Säule zu waschen, und das Elutionsmittel konzentriert wird, um einen Extrakt ohne alkoholischen Geruch zu erhalten;
das makroporöse absorptionsfähige Harz ein makroporöses absorptionsfähiges Harz, ausgewählt aus der Gruppe bestehend aus AB-8, HPD450, HPD700, D101, D4020 oder X5, ist;
in Schritt c) der Extrakt, der in Schritt b) durch Konzentration erhalten wird, mit organischem Lösungsmittel gelöst wird, mit Chromatographiekieselgel gemischt wird, die gut gemischte Probe auf der gut gepackten Kieselgelsäule platziert wird, die Säule mit einer mobilen Phase aus Chloroform : Methanol: Ameisensäure mit einem Volumenverhältnis von 90:10:3-40:10:0,5 eluiert wird.

**4.** Verfahren nach Anspruch 2, **dadurch gekennzeichnet, dass**:

in Schritt a) die Wasserextraktion wie folgt abläuft: Auskochen der Rohdroge mit Wasser mit dem 4-fachen Volumen der Rohdroge für 2 Stunden, Filtern; Auskochen von Drogenrückstand mit Wasser mit dem 3-fachen Volumen des Drogenrückstands für 1 Stunde, Filtern; und Kombinieren des Filtrats, Konzentrieren des Filtrats, um einen Extrakt mit einer relativen Dichte von 1,2 zu erhalten; die Alkoholausfällung wie folgt abläuft: Zugeben von 95-%igem Ethanol zu dem Extrakt zum Ausfällen, bis der Ethanolgehalt 70 % beträgt, und
Stehenlassen für 24 Stunden, Konzentrieren des Überstands durch Rückgewinnen von Ethanol bei Unterdruck und Erhalten eines Extrakts mit einer relativen Dichte von 1,37; in Schritt b) der endgültige Extrakt von Schritt a) auf eine makroporöse absorptionsfähige Harzsäule aufgebracht wird, das Gewichtsverhältnis der Rohdroge zu makroposösem absorptionsfähigem Harz 4:1 beträgt, die Harzsäule mit Wasser mit dem 12-fachen Bettvolumen gewaschen wird, um ein Wasserelutionsmittel zu erhalten, und
Chlorwasserstoffsäure zu dem Wasserelutionsmittel zugegeben wird, um seinen pH-Wert auf 3,0 einzustellen; das saure Elutionsmittel mit dem Gewichtsverhältnis der Rohdroge zu dem makroporösen absorptionsfähigen Harz von 4:1 erneut auf die makroporöse absorptionsfähige Harzsäule aufgebracht wird, die Säule mit Chlorwasserstoffsäure mit einem pH-Wert von 3,0 gewaschen wird, bis das Elutionsmittel nahezu farblos ist; 95-%iges Ethanol mit dem 4-fachen Bettvolumen verwendet wird, um die Säule zu waschen, und das Elutionsmittel konzentriert wird, um einen Extrakt ohne alkoholischen Geruch zu erhalten; das makroporöse absorptionsfähige

Harz AB-8 ist;
in Schritt c) der Extrakt, der in Schritt b) durch Konzentration erhalten wird, mit Methanol gelöst wird, mit 200-300 Mesh-Chromatographiekieselgel gemischt wird, die gut gemischte Probe auf der gut gepackten 200-300 Mesh-Kieselgelsäule platziert wird, die Säule mit einer mobilen Phase aus Chloroform : Methanol: Ameisensäure mit einem Volumenverhältnis von 50:10:2 eluiert wird.

**5.** Verfahren nach einem der Ansprüche 2-4, **dadurch gekennzeichnet, dass** bei der Wasserextraktion in Schritt a) eine wässrige Alkalilösung verwendet wird, wobei das Alkali wenigstens eines, ausgewählt aus der Gruppe bestehend aus Natriumbicarbonat, Natriumcarbonat, Natriumhydroxid, Kaliumbicarbonat, Kaliumcarbonat und Kaliumhydroxid, ist.

**6.** Verfahren nach Anspruch 5, **dadurch gekennzeichnet, dass** die wässrige Alkalilösung eine wässrige Natriumbicarbonatlösung oder eine wässrige Natriumhydroxidlösung ist.

**7.** Verfahren nach Anspruch 6, **dadurch gekennzeichnet, dass** die wässrige Alkalilösung eine wässrige Natriumbicarbonatlösung in einer Konzentration von 0,30 %-0,68 % oder eine wässrige Natriumhydroxidlösung in einer Konzentration von 0,0025 ‰-0,004 ‰ ist.

**8.** Verfahren nach Anspruch 7, **dadurch gekennzeichnet, dass** die wässrige Alkalilösung eine wässrige Natriumbicarbonatlösung in einer Konzentration von 0,45 % ist.

**9.** Verfahren nach einem der Ansprüche 2-8, **dadurch gekennzeichnet, dass** der Schritt a) vor der Wasserextraktion weiterhin eine Alkoholextraktion umfasst.

**10.** Verfahren nach Anspruch 9, wobei die Alkoholextraktion wie folgt abläuft:

zweimaliges Auskochen mit 50-95-%igem Ethanol mit dem 5-8-fachen Volumen der Rohdroge, jeweils 1-2 Stunden, Filtern, Abscheiden der Ethanolextraktionslösung, Extrahieren von Drogenrückstand mit Wasser.

**11.** Pharmazeutische Zusammensetzung, welche die Salvianolsäure L nach Anspruch 1 und pharmazeutisch akzeptable Träger umfasst.

**12.** Verwendung der Salvianolsäure nach Anspruch 1 bei der Herstellung eines Medikaments zum Behandeln von Herz-Kreislauf-Erkrankungen.

**13.** Verwendung nach Anspruch 12, wobei die Herz-Kreislauf-Erkrankung wenigstens eine Erkrankung, ausgewählt aus der Gruppe bestehend aus hypoxiebedingter Vasodilatationsdysfunktion, durch Sauerstoffmangel, Glucosemangel und Überoxidation verursachter *in vitro*-Nervenschädigung und akuter Myokardischämie, ist.

**14.** Verwendung der Salvianolsäure L nach Anspruch 1 bei der Herstellung eines Medikaments, das eine Radikalfangwirkung aufweist.

**15.** Verwendung der Salvianolsäure L nach Anspruch 1 bei der Herstellung eines Medikaments, das eine vorbeugende Antioxidationsfunktionswirkung aufweist.

## Revendications

**1.** Une forme *trans* de l'acide salvianolique L ayant la formule générale (I), ses sels et ses solvates acceptables du point de vue pharmaceutique :

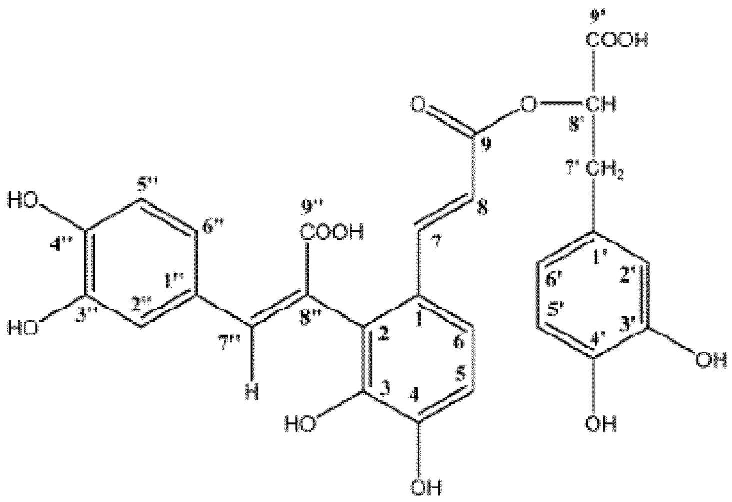

formule (I)

2. Une méthode de préparation de l'acide salvialonique L de la revendication 1, comprenant les étapes suivantes :

a) extraction : procéder à une extraction de la drogue brute *Radix Salviae Miltiorrhizae* ou d'un mélange de *Radix Salviae Miltiorrhizae* et d'autres drogues brutes avec de l'eau, en ajoutant de l'alcool pour précipiter et obtenir un surnageant, puis concentrer le surnageant pour obtenir un extrait ;
b) séparation : dissoudre l'extrait de l'étape a) dans de l'eau, appliquer sur une résine absorbante macroporeuse puis éluer la résine avec de l'eau afin d'obtenir un éluant, acidifier l'éluant, appliquer de nouveau l'éluant acidfié sur la résine absorbante macroporeuse, laver la résine à l'aide d'une solution aqueuse acide afin d'ôter les impuretés, puis éluer la résine à l'aide d'éthanol afin d'obtenir un éluant d'éthanol, concentrer l'éluant d'éthanol afin d'obtenir un extrait ;
c) purification : appliquer l'extrait de l'étape b) sur une colonne de gel de silice, procéder à une élution isocratique avec une phase mobile de chloroforme, de méthanol et d'acide formique ; recueillir l'éluant ; surveiller l'ensemble du processus d'élution par CCM, combiner les éluants typiquement analogues afin d'obtenir l'acide salvianolique L.

3. La méthode selon la revendication 2, **caractérisée en ce que** : au cours de l'étape a), ladite drogue crue *Radix Salviae Miltiorrhizae* ou le mélange de *Radix Salviae Miltiorrhizae* et d'autres drogues crues sont découpés en éléments de décoction ; ladite extraction à l'eau est comme suit : procéder à la décoction de la drogue crue avec un volume d'eau égal à 4 à 8 fois le volume de la drogue crue pendant 1,5 à 3,5 heures, filtrer ; procéder à la décoction du résidu de drogue avec un volume d'eau égal à 3 à 6 fois le volume du résidu de drogue pendant 1-3 heures, filtrer ; et combiner le filtrat, concentrer le filtrat afin d'obtenir un extrait avec une densité relative de 1,11-1,28 (80 °C) ; ladite précipitation alcoolique est comme suit : ajouter à l'extrait de l'éthanol à 95% afin de précipiter jusqu'à ce que le contenu de l'éthanol soit de 65%-70% et laisser reposer pendant 12 à 36 heures, concentrer le surnageant en récupérant l'éthanol sous des conditions de pression réduites, et obtenir un extrait d'une densité relative de 1,30-1,38 (60°C) ;
au cours de l'étape b), l'extrait final de l'étape (a) est appliqué à une colonne de résine absorbante macroporeuse, le rapport de poids entre la drogue crue et la résine absorbante macroporeuse est de 5:1-1:1, la colonne de résine est lavée avec un volume d'eau égal à 8 à 15 fois le volume du lit afin d'obtenir un éluant aqueux, et de l'acide chlorhydrique est ajouté à l'éluant aqueux afin d'ajuster la valeur du pH à 2,2-3,5 ; ledit éluant acide est appliqué de nouveau à la colonne de résine absorbante macroporeuse, avec le rapport de poids entre la drogue crue et la résine absorbante macroporeuse de 5:1-1:1, la colonne est lavée avec de l'acide chlorhydrique ayant une valeur de pH de 2,2-3,5 jusqu'à ce que l'éluant soit presque incolore ; un volume d'éthanol à 50%-95% égal à 3 à 8 fois le volume du lit est utilisé afin de laver la colonne, et l'éluant est concentré afin d'obtenir un extrait sans odeur d'alcool ; ladite résine absorbante macroporeuse est une résine absorbante macroporeuse sélectionnée parmi le groupe se composant de AB-8, HPD450, HPD700, D101, D4020 ou X5;
au cours de l'étape c), l'extrait obtenu par concentration au cours de l'étape b) est dissous avec du solvant organique, mélangé avec du gel de silice chromatographique, l'échantillon bien mélangé est placé dans une colonne de gel

de silice bien tassée, la colonne est éluée avec une phase mobile de chloroforme : méthanol : acide formique, avec un rapport volumique de 90:10:3-40:10:0,5.

4. La méthode selon la revendication 2, **caractérisée en ce que** : au cours de l'étape a), ladite extraction à l'eau est comme suit : procéder à la décoction de la drogue crue avec un volume d'eau égal à 4 fois le volume de la drogue crue pendant 2 heures, filtrer ; procéder à la décoction du résidu de drogue avec un volume d'eau égal à 3 fois le volume du résidu de drogue pendant 1 heure, filtrer ; et combiner le filtrat, concentrer le filtrat afin d'obtenir un extrait avec une densité relative de 1,2 ; ladite précipitation alcoolique est comme suit :

    ajouter à l'extrait de l'éthanol à 95 % afin de précipiter jusqu'à ce que le contenu de l'éthanol soit de 70% et laisser reposer pendant 24 heures, concentrer le surnageant en récupérant l'éthanol sous des conditions de pression réduites, et obtenir un extrait d'une densité relative de 1,37 ;
    Au cours de l'étape b), l'extrait final de l'étape (a) est appliqué à la colonne de résine absorbante macroporeuse, le rapport de poids entre la drogue crue et la résine absorbante est de 4:1, la colonne de résine est lavée avec un volume d'eau égal à 12 fois le volume du lit afin d'obtenir un éluant aqueux, et de l'acide chlorhydrique est ajouté à l'éluant aqueux afin d'ajuster la valeur du pH à 3,0 ; ledit éluant acide est appliqué de nouveau à la colonne de résine absorbante macroporeuse, avec le rapport de poids entre la drogue crue et la résine absorbante macroporeuse de 4:1, la colonne est lavée avec de l'acide chlorhydrique ayant une valeur de pH de 3,0 jusqu'à ce que l'éluant soit presque incolore ; un volume d'éthanol à 95% égal à 4 fois le volume du lit est utilisé afin de laver la colonne, et l'éluant est concentré afin d'obtenir un extrait sans odeur d'alcool ; ladite résine absorbante macroporeuse est AB-8;
    Au cours de l'étape c), l'extrait obtenu par concentration au cours de l'étape b) est dissous avec du méthanol, mélangé avec du gel de silice chromatographique 200-300 mesh, l'échantillon bien mélangé est placé dans une colonne de gel de silice 200-300 mesh bien tassée, la colonne est éluée avec une phase mobile de chloroforme : méthanol : acide formique, avec un rapport volumique de 50:10:2.

5. La méthode selon l'une quelconque des revendications 2 à 4, **caractérisée en ce qu'**une solution aqueuse alcaline est utilisée dans ladite extraction à l'eau au cours de l'étape a), ledit alcali étant au moins un sélectionné parmi le groupe se composant de bicarbonate de sodium, de carbonate de sodium, d'hydroxyde de sodium, de bicarbonate de potassium, de carbonate de potassium et d'hydroxyde de potassium.

6. La méthode selon la revendication 5, **caractérisée en ce que** ladite solution aqueuse alcaline est une solution aqueuse de bicarbonate de sodium ou une solution aqueuse d'hydroxyde de sodium.

7. La méthode selon la revendication 6, **caractérisée en ce que** ladite solution aqueuse alcaline est une solution aqueuse de bicarbonate de sodium à une concentration de 0,30%-0,68% ou une solution aqueuse d'hydroxyde de sodium à une concentration de 0,0025‰-0,004‰.

8. La méthode selon la revendication 7, **caractérisée en ce que** ladite solution aqueuse alcaline est une solution aqueuse de bicarbonate de sodium à une concentration de 0,45%.

9. La méthode selon l'une quelconque des revendications 2 à 8, **caractérisée en ce que** l'étape a) comprend de plus une extraction à l'alcool avant l'extraction à l'eau.

10. La méthode selon la revendication 9, ladite extraction à l'alcool étant comme suit :

    procéder à la décoction d'un volume d'éthanol à 50-95% égal à 5 à 8 fois le volume de la drogue crue, de 1 à 2 heures chaque fois, filtrer, éliminer la solution d'extraction à l'éthanol, extraire le résidu de drogue avec de l'eau.

11. Une composition pharmaceutique comprenant ledit acide salvianolique L de la revendication 1 et des supports acceptables du point de vue pharmaceutique.

12. Une utilisation dudit acide salvianolique L de la revendication 1 dans la préparation d'un médicament pour le traitement des maladies cardiovasculaires.

13. L'utilisation selon la revendication 12, ladite maladie cardiovasculaire étant au moins une maladie sélectionnée parmi le groupe se composant du dysfonctionnement de vasodilatation par hypoxie, une lésion neuronale *in vitro* entraînée par un manque d'oxygène, un manque et de glucose et un état de suroxidation, et l'ischémie myocardique

aiguë.

14. Une utilisation dudit acide salvianolique L de la revendication 1 dans la préparation d'un médicament ayant une activité de piégeage des radicaux libres.

15. Une utilisation dudit acide salvianolique L de la revendication 1 dans la préparation d'un médicament ayant une activité de fonction anti-oxydante préventive.

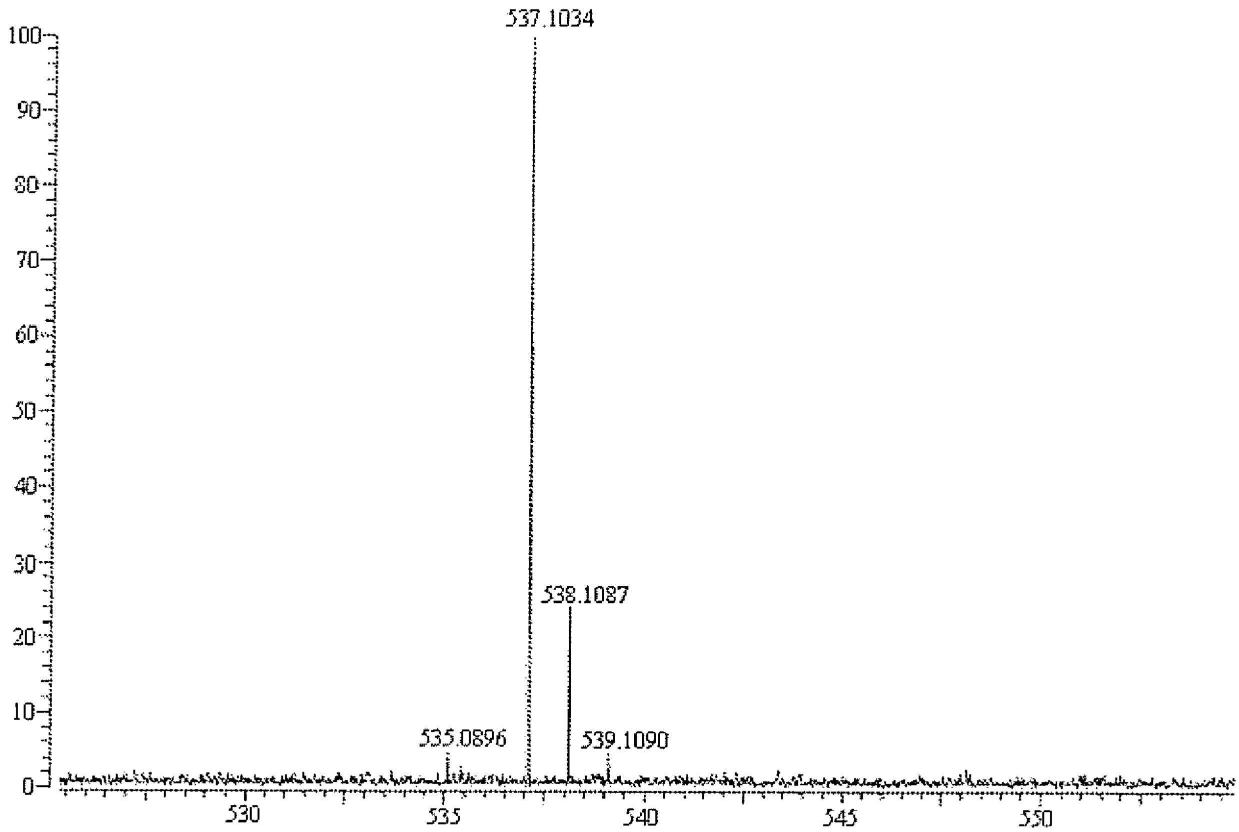
537.1034
538.1087
535.0896
539.1090
100
90
80
70
60
50
40
30
20
10
0
530
535
540
545
550

FIG.1

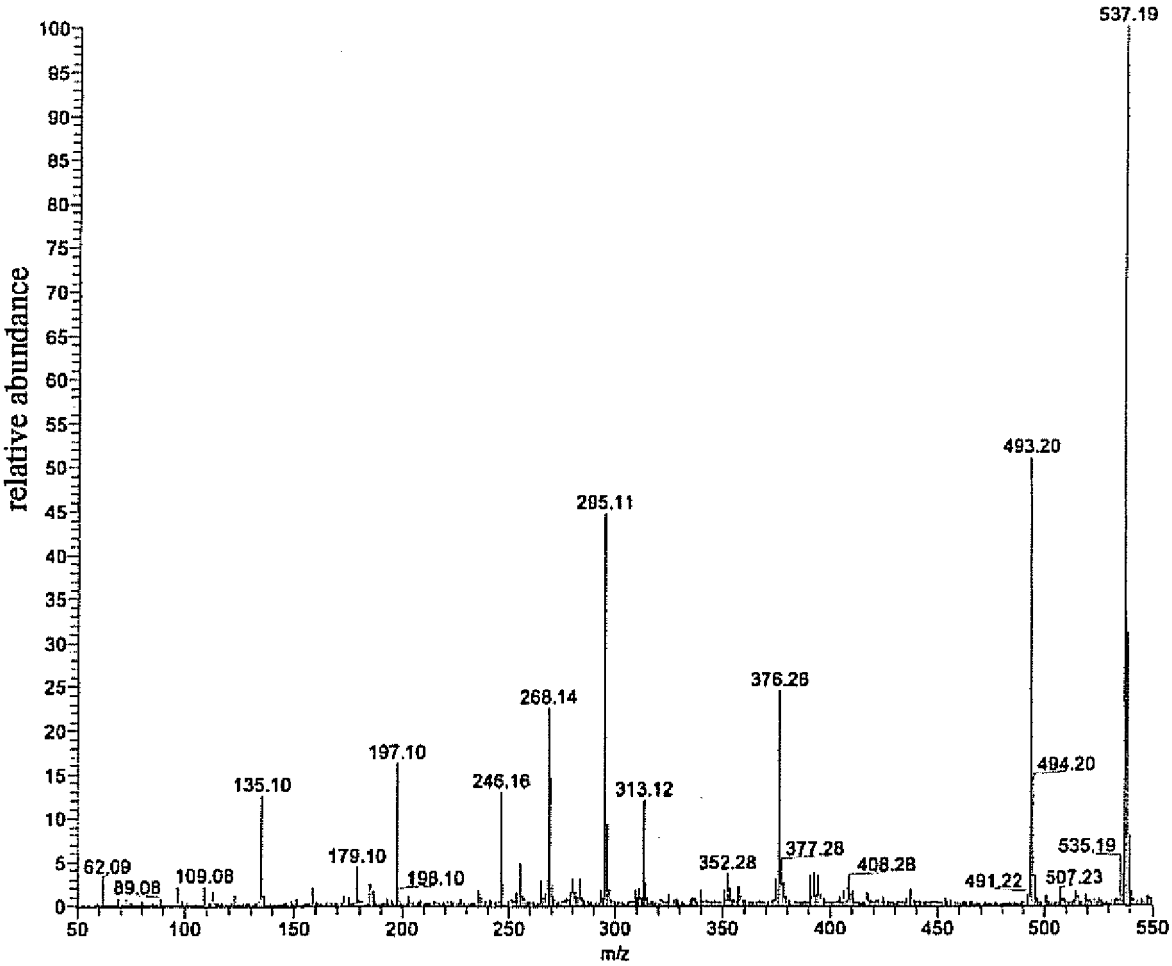
537.19
493.20
285.11
376.28
268.14
197.10
494.20
135.10
246.16
313.12
535.19
179.10
352.28
377.28
408.28
62.09
89.08
109.08
198.10
491.22
507.23
relative abundance
m/z

FIG.2

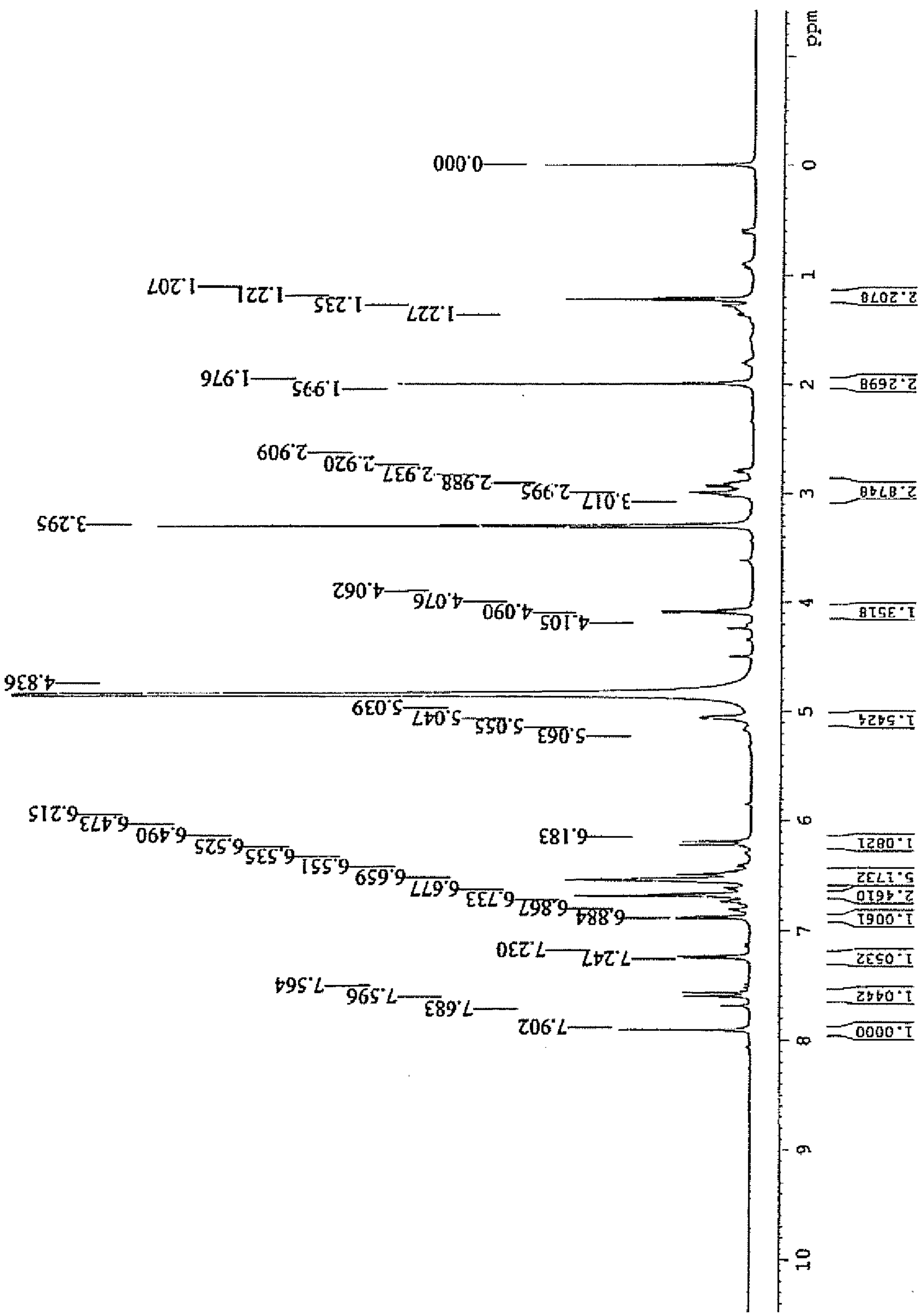
ppm
0.000
1.207
1.221
1.235
1.227
1.976
1.995
2.909
2.920
2.937
2.988
2.995
3.017
3.295
4.062
4.076
4.090
4.105
4.836
5.039
5.047
5.055
5.063
6.215
6.473
6.490
6.525
6.535
6.551
6.659
6.677
6.733
6.867
6.884
6.183
7.230
7.247
7.564
7.596
7.683
7.902
2.2078
2.2698
2.8748
1.3518
1.5424
1.0821
5.1732
2.4610
1.0061
1.0532
1.0442
1.0000

FIG.3

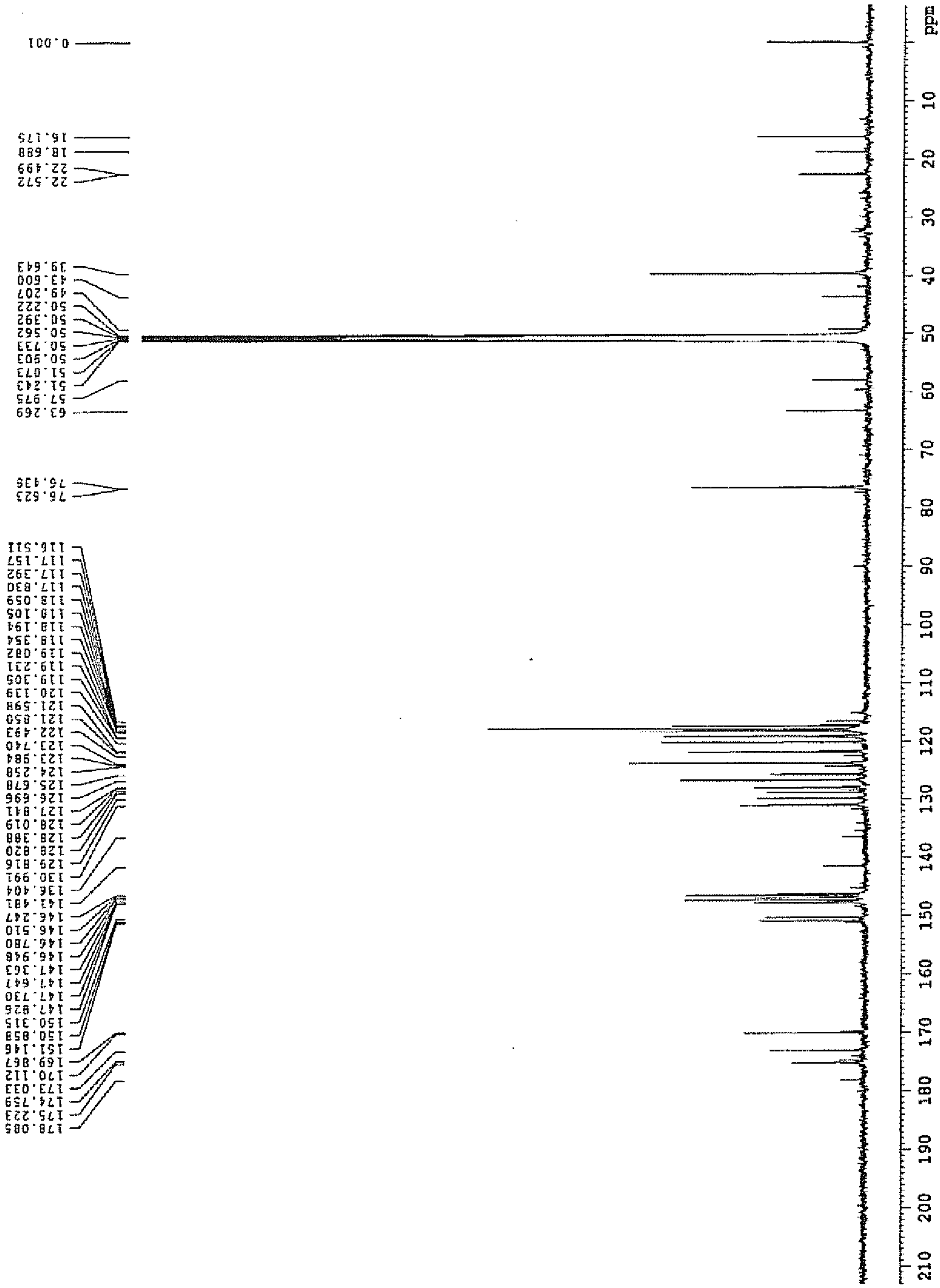
0.001
16.175
18.688
22.499
22.572
39.643
43.600
49.207
50.222
50.392
50.562
50.733
50.903
51.073
51.243
57.975
63.269
76.435
76.623
116.511
117.157
117.392
117.830
118.059
118.105
118.194
118.354
119.082
119.221
119.305
120.139
121.598
121.850
122.493
123.740
123.984
124.258
125.678
126.696
127.841
128.019
128.388
128.820
129.816
130.991
136.404
141.481
146.247
146.510
146.780
146.948
147.363
147.647
147.730
147.826
150.315
150.858
151.146
169.867
170.112
173.033
174.759
175.223
178.085
ppm
10
20
30
40
50
60
70
80
90
100
110
120
130
140
150
160
170
180
190
200
210

FIG.4

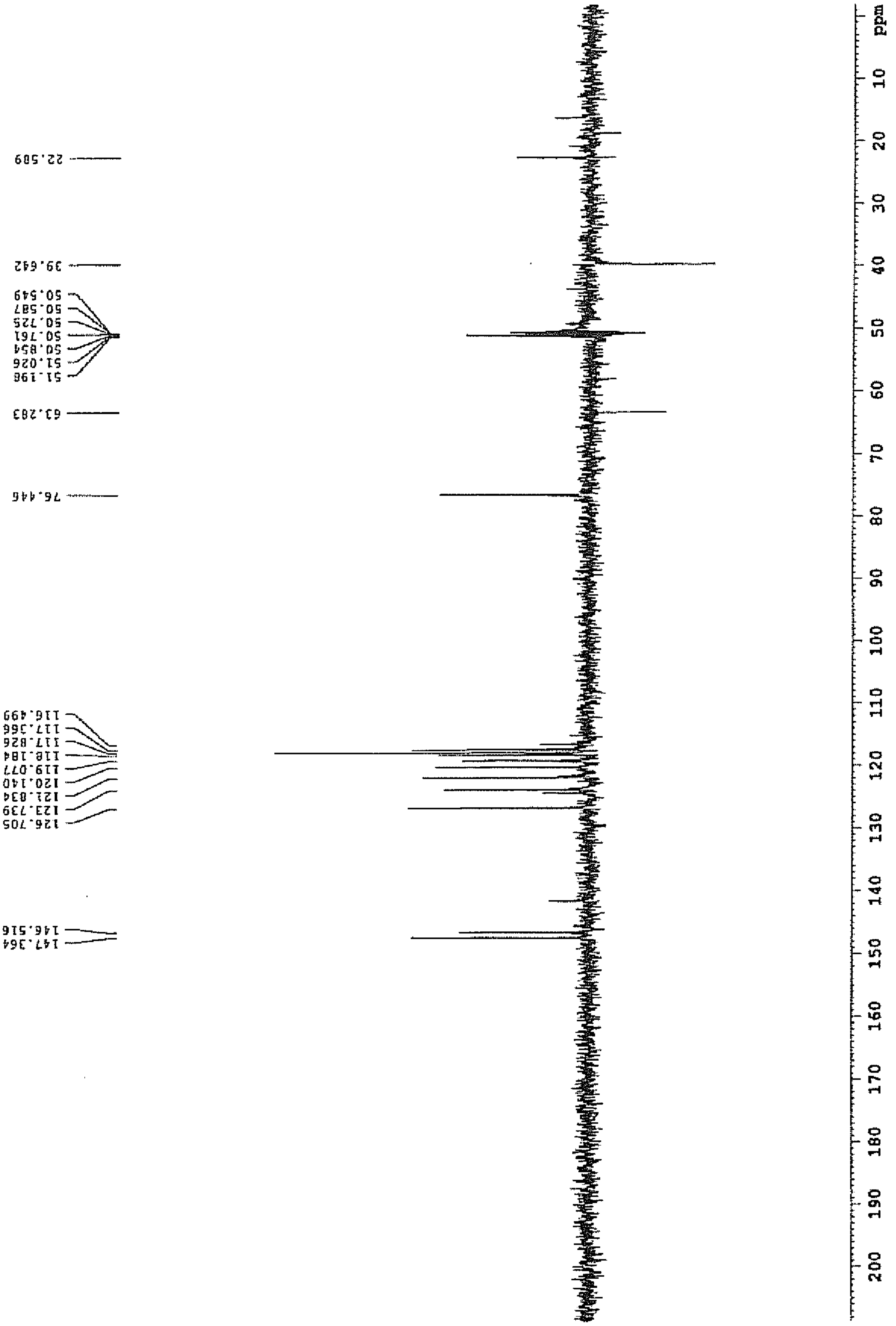
22.589
39.642
50.549
50.587
50.725
50.761
50.854
51.026
51.198
63.283
76.446
116.499
117.366
117.826
118.184
119.077
120.140
121.834
123.739
126.705
146.516
147.364
ppm
10
20
30
40
50
60
70
80
90
100
110
120
130
140
150
160
170
180
190
200

FIG.5

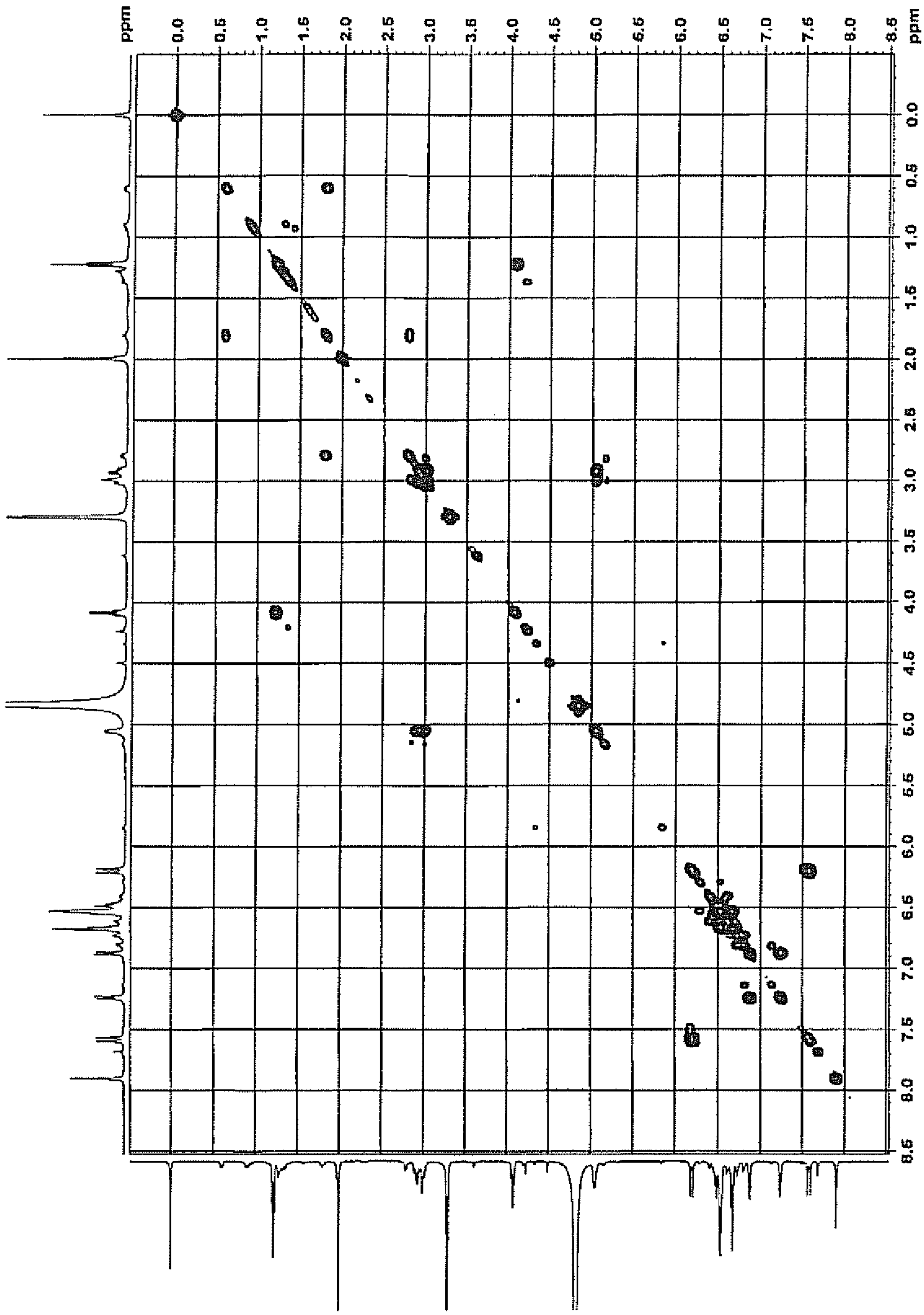

FIG.6

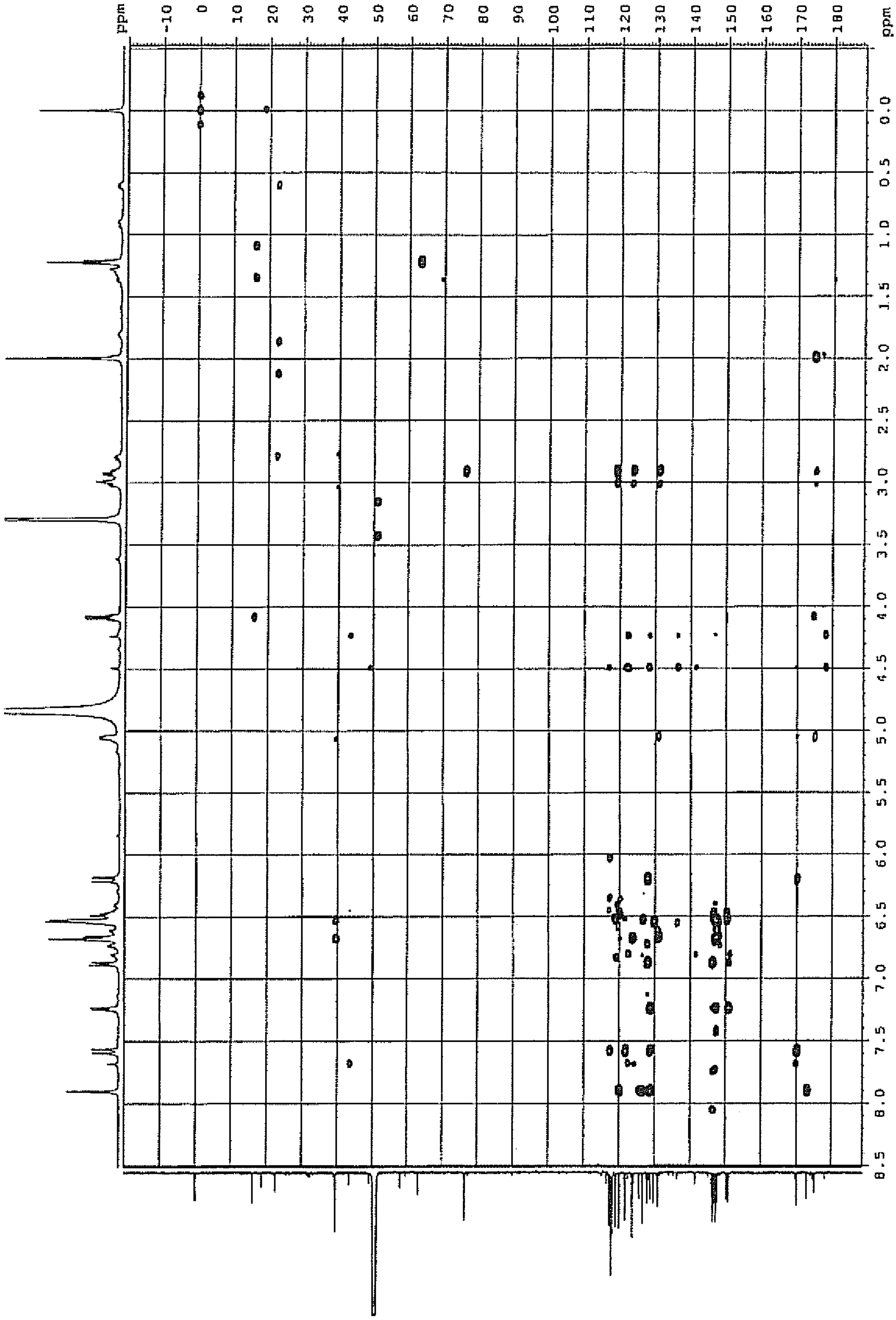
ppm
-10
0
10
20
30
40
50
60
70
80
90
100
110
120
130
140
150
160
170
180
ppm
0.0
0.5
1.0
1.5
2.0
2.5
3.0
3.5
4.0
4.5
5.0
5.5
6.0
6.5
7.0
7.5
8.0
8.5

FIG.7

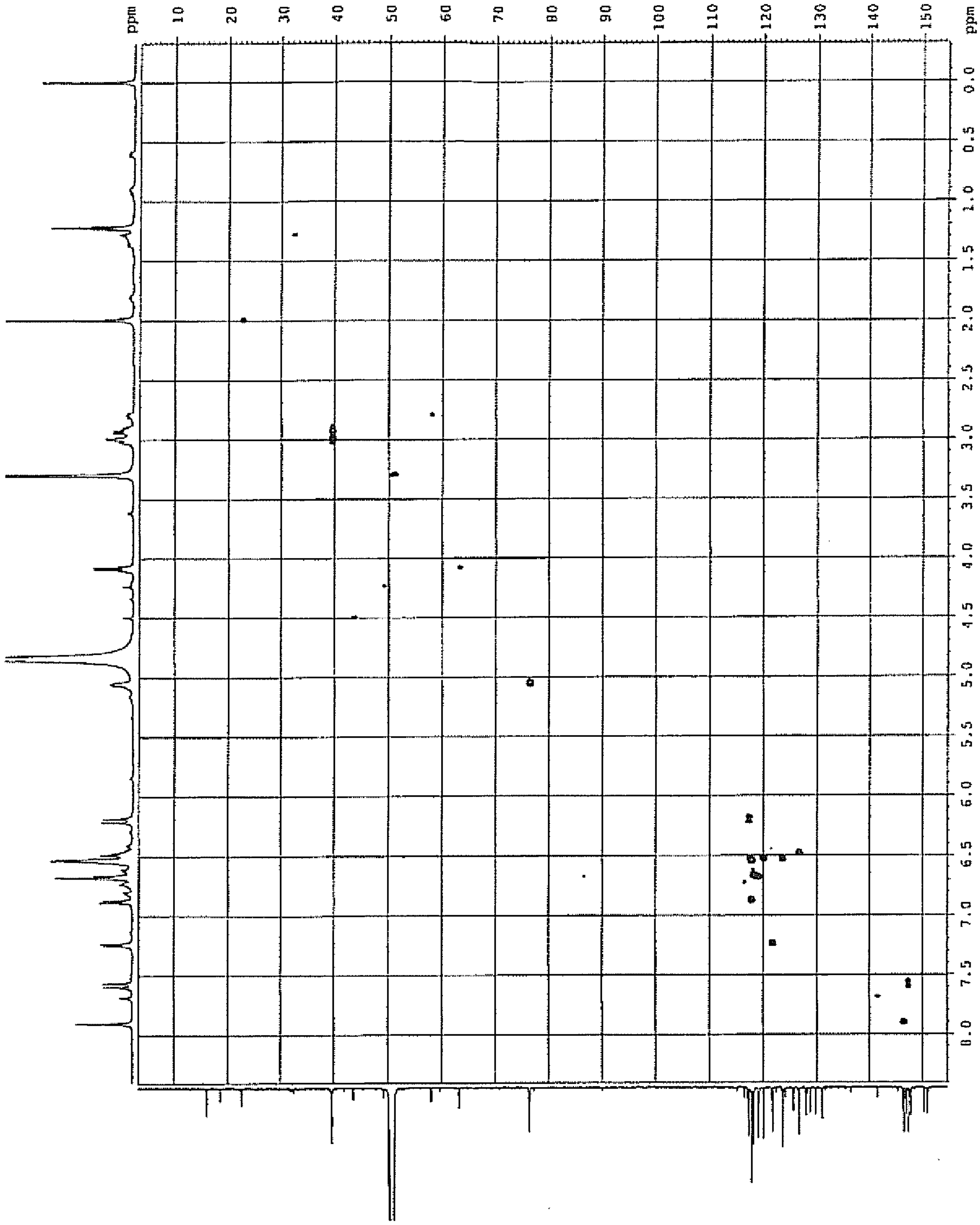
ppm
10
20
30
40
50
60
70
80
90
100
110
120
130
140
150
ppm
0.0
0.5
1.0
1.5
2.0
2.5
3.0
3.5
4.0
4.5
5.0
5.5
6.0
6.5
7.0
7.5
8.0

FIG.8

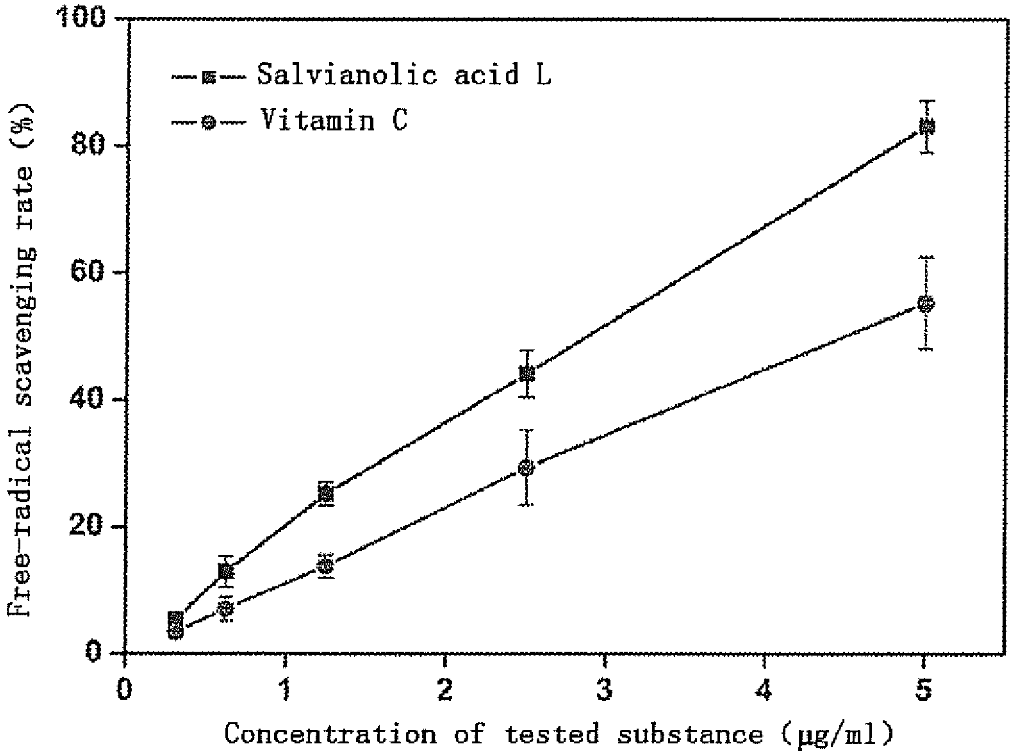
100
80
60
40
20
0
0
1
2
3
4
5
Salvianolic acid L
Vitamin C
Free-radical scavenging rate (%)
Concentration of tested substance (μg/ml)

FIG.9

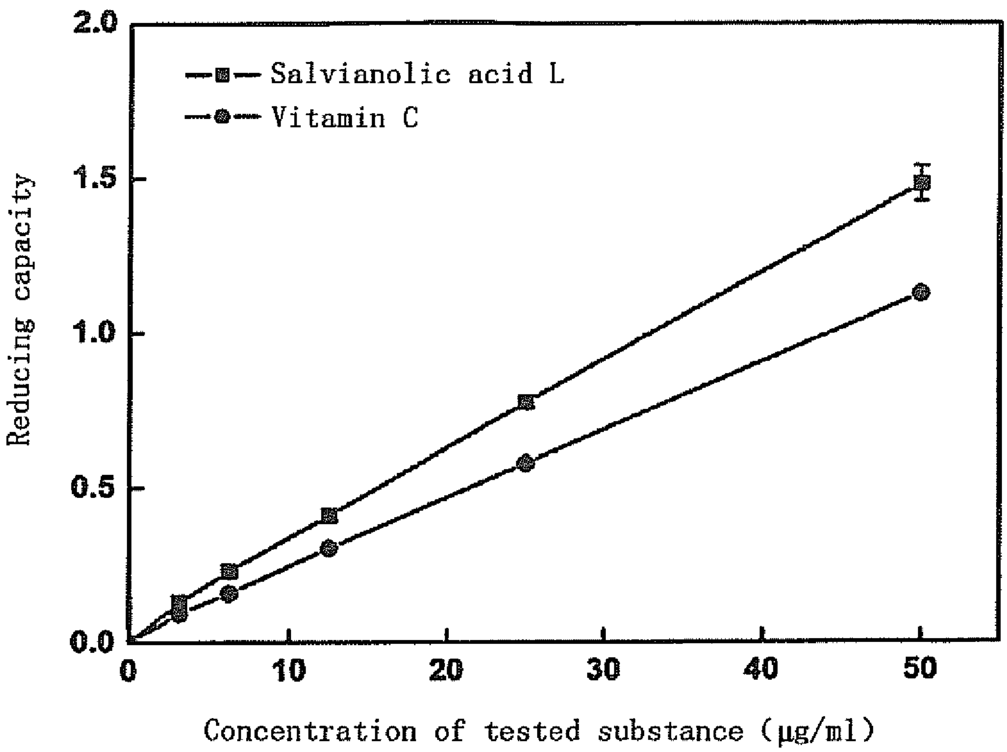
2.0
1.5
1.0
0.5
0.0
Reducing capacity
Salvianolic acid L
Vitamin C
0
10
20
30
40
50
Concentration of tested substance (μg/ml)

FIG.10

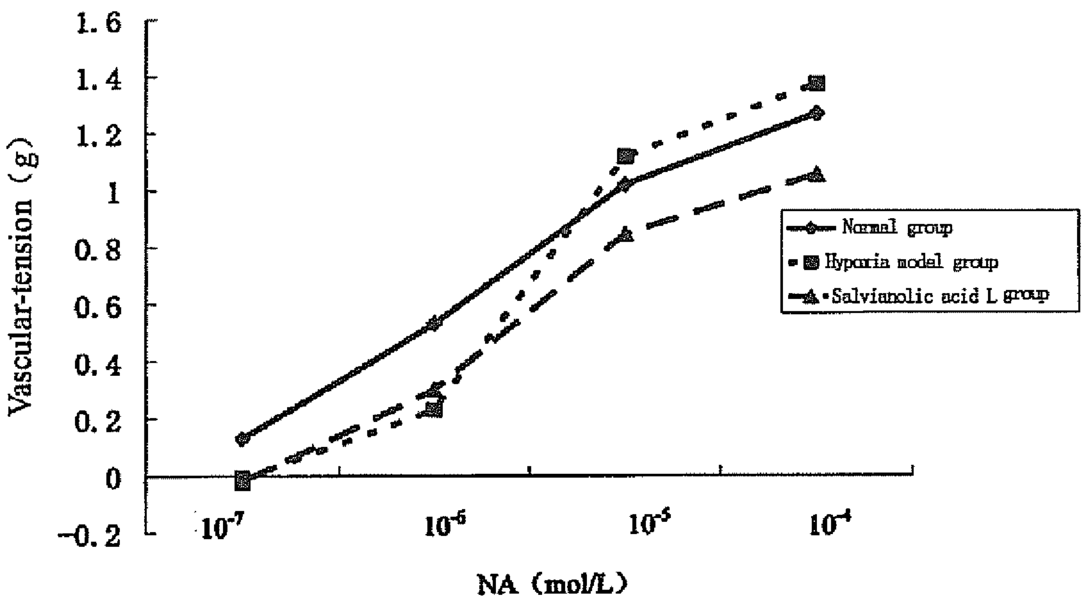
1.6
1.4
1.2
1
0.8
0.6
0.4
0.2
0
-0.2
Vascular-tension (g)
Normal group
Hyporia model group
Salvianolic acid L group
10-7
10-6
10-5
10-4
NA (mol/L)

FIG.11

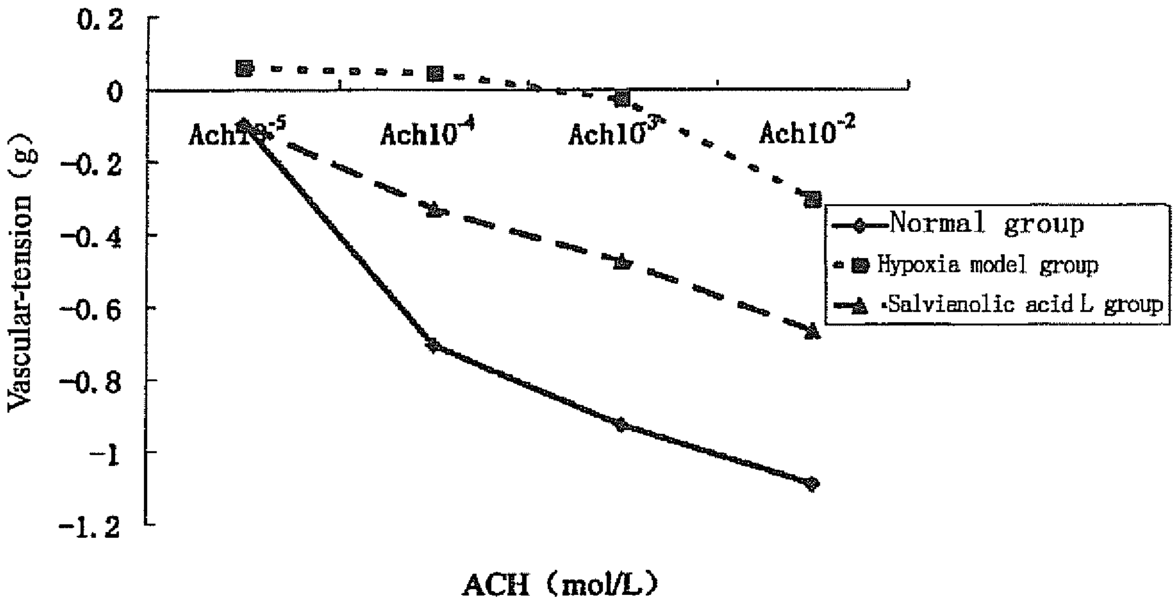
0.2
0
-0.2
-0.4
-0.6
-0.8
-1
-1.2
Vascular-tension (g)
Ach10-5
Ach10-4
Ach10-3
Ach10-2
Normal group
Hypoxia model group
Salvianolic acid L group
ACH (mol/L)

FIG.12

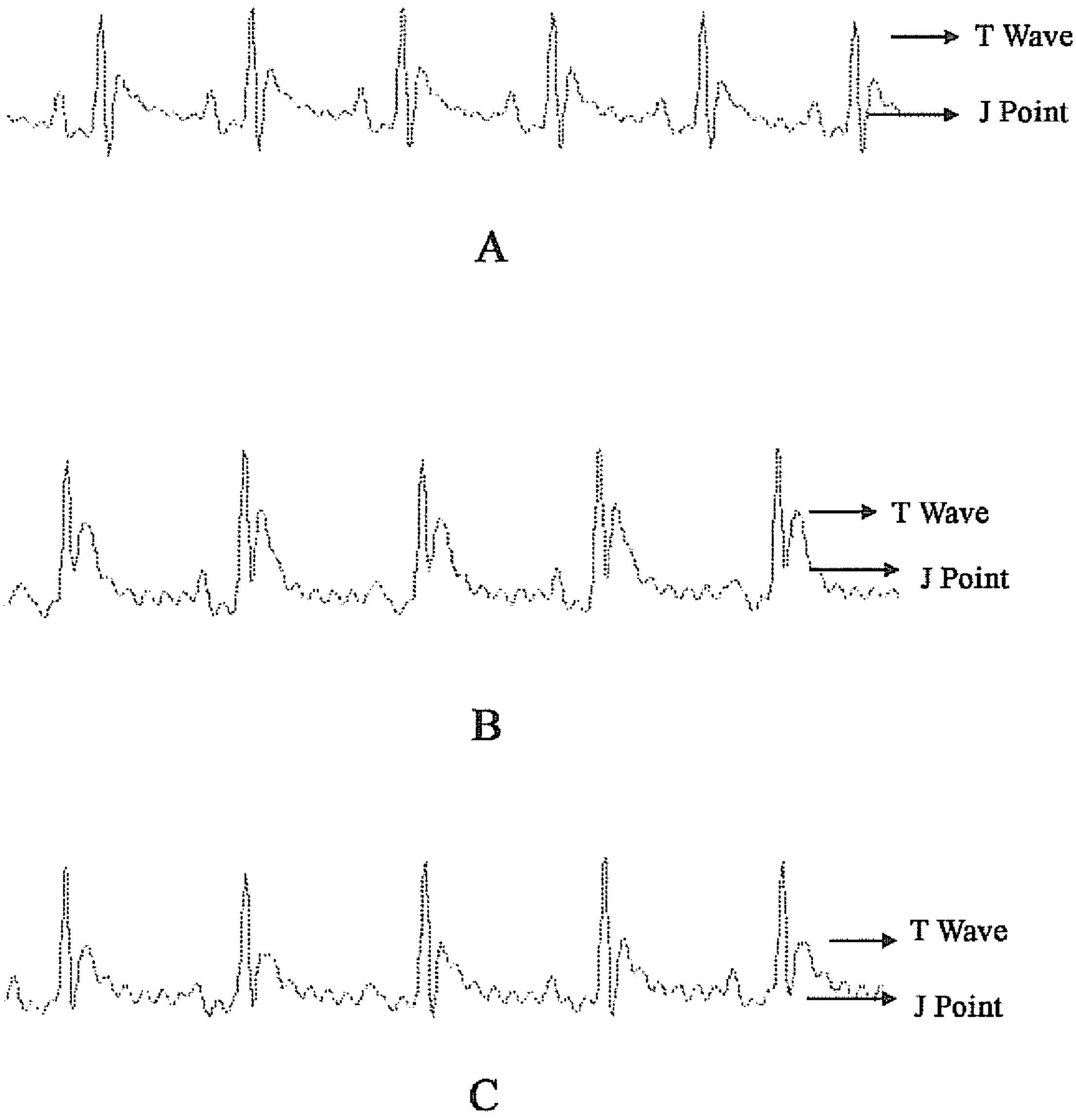


FIG.13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1741439 A1


### Non-patent literature cited in the description


- **L.N. LI et al.** Institute of Materia Medica, Chinese Academy of Medical Sciences & Peking Union Medical College. *Bulletin of Medical Research,* 2001, vol. 30 (7
- **RENA. KASIMU et al.** *Journal of Xinjiang Medical University,* 2002, vol. 25 (3